# EUROPEAN PATENT SPECIFICATION

(11) **EP 2 104 716 B1**
(45) Date of publication and mention of the grant of the patent: **21.02.2018**
(21) Application number: 07857654.3
(22) Date of filing: 17.12.2007
(51) Int. Cl.: C09D 7/12, C08K 5/3492, C07D 251/24, C09K 15/30

(54) **TINTED CLEAR COATINGS UV STABILIZED WITH 2-HYDROXY PHENYL TRIAZINE**
GETÖNTE KLARE BESCHICHTUNGEN UV STABILISIERT MIT 2-HYDROXY PHENYL TRIAZIN
REVÊTEMENTS CLAIRS TEINTÉS STABILISÉ PAR 2-HYDROXY PHENYL TRIAZINE

(30) Priority: 15.01.2007 EP 07100511
(43) Date of publication of application: 30.09.2009
(73) Proprietor: BASF SE, 67056 Ludwigshafen (DE)
(72) Inventor: JAHN, Reiner, 79379 Müllheim (DE); HENKE, Daniel, CH-4057 Basel (CH); BRAIG, Adalbert, 79589 Binzen (DE)
(74) Representative: BASF IP Association
(86) International application number: PCT/EP2007/064020
(87) International publication number: WO 2008/086929

(56) References cited:
- EP-A2- 0 148 718
- WO-A-01/47900
- WO-A-03/086661
- WO-A-2006/131469
- US-A- 5 686 233
- US-A- 6 060 543
- US-A1- 2004 028 823

## Description

The present invention pertains to tinted clear coating compositions comprising (a) a 2-hydroxy phenyl triazine UV absorber, (b) a pigment, and (c) a binder and to coatings obtained by applying such compositions to a substrate.

The tinted clear coatings of the invention may show an increased chroma, an increased color brilliance and a higher durability especially if compared to untinted clear coatings. Moreover these tinted clear coatings may show a decreased color deviation after weathering, especially if compared to untinted clear coatings.

WO03/086661 discloses a coloured base coat and a clear coat, wherein the clear coat comprises effect pigments and a 2-hydroxy phenyl triazine UV absorber. WO2006/131469 discloses a non-pigmented clear coating composition comprising a hydroxy phenyl triazine UV absorber for automotive application. US2004028823 describes a new tricoat process as well as conventional processes for basecoat-clearcoat compositions for automotive application.

The present invention pertains to a tinted clear coating composition comprising
(a) at least one 2-hydroxy phenyl triazine UV absorber,
(b) at least one pigment selected from the group consisting of quinacridone, diketo-pyrrolo-pyrrole, quinacridone/diketo-pyrrolo-pyrrole, isoindolinone, phthalocyanine, perylene, anthraquinone Red, indanthrone Blue, azomethine Cu complex, mono azo Ni complex, quinophthalone, isoindoline and naphthol AS and mixtures (e.g. crystal mixtures) and solid solutions thereof, wherein the ratio of component (a) to component (b) is from 10:1 to 1:1 by weight, preferably from 6:1 to 1.3:1, more preferably from 5:1 to 1.5:1, most preferably from 4:1 to 2:1, by weight. and
(c) at least one binder.

These pigment mixtures and solid solutions (b) are to be understood to include mixtures such as crystal mixtures and solid solutions of two or more diketo-pyrrolo-pyrrole pigments, of two or more quinacridone pigments and of two and more quinacridone/diketo-pyrrolo-pyrrole pigments.

A tinted clear coating composition is to be understood that when applied to a substrate, the tinted clear coating is neither completely transparent and colourless as a clear coating nor completely opaque as a typical pigmented coating. A tinted clear coating is transparent and coloured or semi-transparent and coloured.

For instance, the 2-hydroxy phenyl triazine UV absorber (a) is of formula (I), (II), (III), (IV), (V) or (VI), preferably of formula (I), (II) or (III) 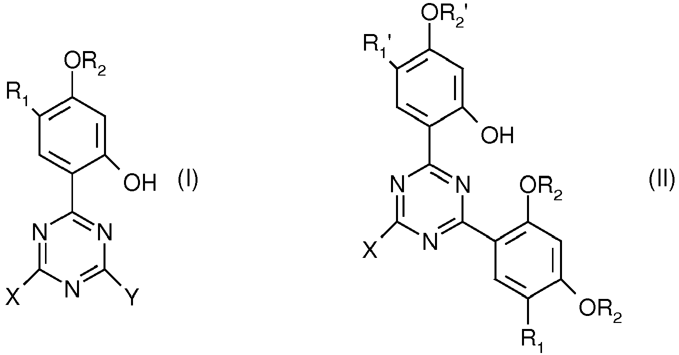 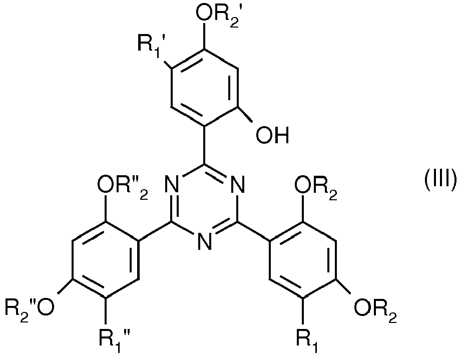 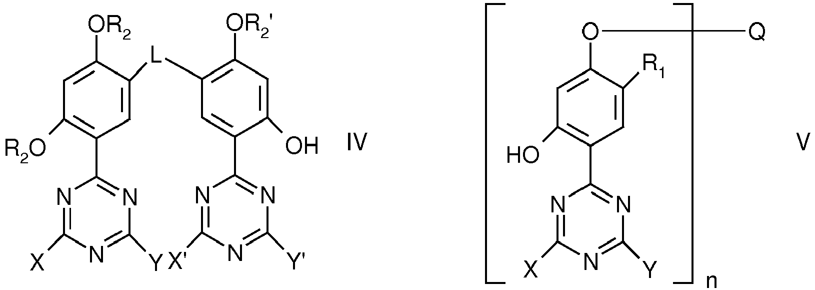 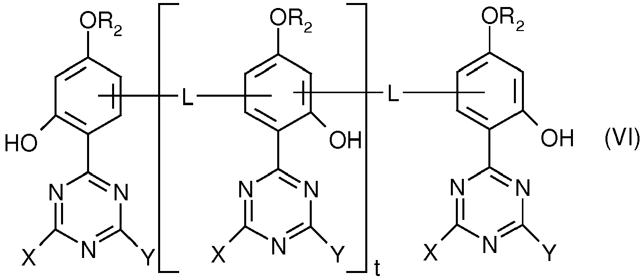 wherein
X and Y are independently phenyl, naphthyl, pyrenyl, phenanthrenyl or fluoranthenyl, or said phenyl, said naphthyl, said pyrenyl, said phenanthrenyl or said fluoranthenyl substituted by one to three alkyl of 1 to 6 carbon atoms, by halogen, by hydroxy or by alkoxy of 1 to 6 carbon atoms or by mixtures thereof; or are independently Z₁ or Z₂;
X, X', Y and Y' are the same or different and are as defined for X and Y;
R₁ is hydrogen, straight or branched chain alkyl of 1 to 24 carbon atoms, cycloalkyl of 5 to 12 carbon atoms, phenylalkyl of 7 to 15 carbon atoms, halogen, -SR₃, -SOR₃ or -SO₂R₃; or said alkyl, said cycloalkyl or said phenylalkyl substituted by one to three halogen, -R₄, -OR₅, -N(R₅)₂, -COR₅, -COOR₅, -OCOR₅, -CN, -NO₂, -SR₅, -SOR₅, -SO₂R₅ or -P(O)(OR₅)₂, morpholinyl, piperidinyl, 2,2,6,6-tetramethylpiperidinyl, piperazinyl or N-methylpiperidinyl groups or combinations thereof; or said alkyl or said cycloalkyl interrupted by one to four phenylene, -O-, -NR₅-, -CONR₅-, -COO-, -OCO- or -CO groups or combinations thereof; or said alkyl or said cycloalkyl both substituted and interrupted by combinations of the groups mentioned above;
R₁, R₁' and R₁" are the same or different and are as defined for R₁;
R₂ is hydrogen, straight or branched chain alkyl of 1 to 24 carbon atoms or cycloalkyl of 5 to 12 carbon atoms; or said alkyl or said cycloalkyl substituted by one to four halogen, epoxy, glycidyloxy, furyloxy, -R₄, -OR₅, -N(R₅)₂, -CON(R₅)₂, -COR₅, -COOR₅, -OCOR₅, -OCOC(R₅)=C(R₅)₂, -C(R₅)=CCOOR₅, -CN, -NCO, or 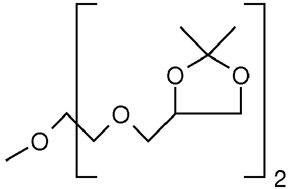 or combinations thereof; or said alkyl or said cycloalkyl interrupted by one to four epoxy, -O-, -NR₅-, -CONR₅-, -COO-, -OCO-, -CO-, -C(R₅)=C(R₅)COO-, -OCOC(R₅)=C(R₅)-, - C(R₅)=C(R₅)-, phenylene or phenylene-G-phenylene in which G is -O-, -S-, -SO₂-, -CH₂- or - C(CH₃)₂- or combinations thereof, or said alkyl or said cycloalkyl both substituted and interrupted by combinations of the groups mentioned above; or R₂ is -SO₂R₃ or -COR₆;
R₂, R₂' and R₂" are the same or different and are as defined for R₂;
R₃ is alkyl of 1 to 20 carbon atoms, alkenyl of 3 to 18 carbon atoms, cycloalkyl of 5 to 12 carbon atoms, phenylalkyl of 7 to 15 carbon atoms, aryl of 6 to 10 carbon atoms or said aryl substituted by one or two alkyl of 1 to 4 carbon atoms;
R₄ is aryl of 6 to 10 carbon atoms or said aryl substituted by one to three halogen, alkyl of 1 to 8 carbon atoms, alkoxy of 1 to 8 carbon atoms or combinations thereof; cycloalkyl of 5 to 12 carbon atoms; phenylalkyl of 7 to 15 carbon atoms or said phenylalkyl substituted on the phenyl ring by one to three halogen, alkyl of 1 to 8 carbon atoms, alkoxy of 1 to 8 carbon atoms or combinations thereof; or straight or branched chain alkenyl of 2 to 18 carbon atoms; R₅ is defined as is R₄; or R₅ is also hydrogen or straight or branched chain alkyl of 1 to 24 carbon atoms, alkenyl of 2 to 24 carbon atoms; or R₅ is a group for formula 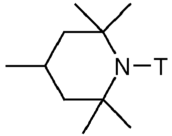
T is hydrogen, oxyl, hydroxyl, -OT₁, alkyl of 1 to 24 carbon atoms, said alkyl substituted by one to three hydroxy; benzyl or alkanoyl of 2 to 18 carbon atoms;
T₁ is alkyl of 1 to 24 carbon atoms, cycloalkyl of 5 to 12 carbon atoms, alkenyl of 2 to 24 carbon atoms, cycloalkenyl of 5 to 12 carbon atoms, phenylalkyl of 7 to 15 carbon atoms, a radical of a saturated or unsaturated bicyclic or tricyclic hydrocarbon of 7 to 12 carbon atoms or aryl of 6 to 10 carbon atoms or said aryl substituted by one to three alkyl of 1 to 4 carbon atoms;
R₆ is straight or branched chain alkyl of 1 to 18 carbon atoms, straight or branched chain alkenyl of 2 to 12 carbon atoms, phenoxy, alkylamino of 1 to 12 carbon atoms, arylamino of 6 to 12 carbon atoms, -R₇COOH or -NH-R₈-NCO;
R₇ is alkylene of 2 to 14 carbon atoms or phenylene;
R₈ is alkylene of 2 to 24 carbon atoms, phenylene, tolylene, diphenylmethane or a group 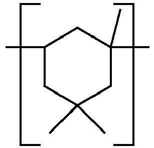
t is 0 to 9;
L is straight or branched alkylene of 1 to 12 carbon atoms, cycloalkylene of 5 to 12 carbon atoms or alkylene substituted or interrupted by cyclohexylene or phenylene; or L is benzylidene; or L is -S-, -S-S-, -S-E-S-, -SO-, -SO₂-, -SO-E-SO-, -SO₂-E-SO₂-, -CH₂-NH-E-NH-CH₂- or 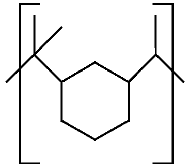
E is alkylene of 2 to 12 carbon atoms, cycloalkylene of 5 to 12 carbon atoms or alkylene interrupted or terminated by cycloalkylene of 5 to 12 carbon atoms; n is 2, 3 or 4;
when n is 2; Q is straight or branched alkylene of 2 to 16 carbon atoms; or said alkylene substituted by one to three hydroxy groups; or said alkylene interrupted by one to three -CH=CH- or -O-; or said alkylene both substituted and interrupted by combinations of the groups mentioned above; or Q is xylylene or a group -CONH-R₈-NHCO-, - CH₂CH(OH)CH₂O-R₉-OCH₂CH(OH)CH₂-, -CO-R₁₀-CO-, or -(CH₂)ₘ-COO-R₁₁-OOC-(CH₂)ₘ-, where m is 1 to 3; or Q is 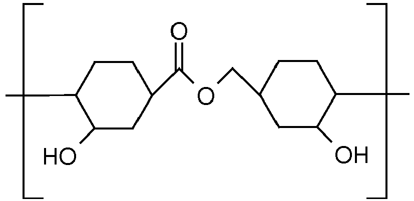
R₉ is alkylene of 2 to 50 carbon atoms; or said alkylene interrupted by one to ten -O-, phenylene or a group -phenylene-G-phenylene in which G is -O-, -S-, -SO₂-, -CH₂- or -C(CH₃)₂-;
R₁₀ is alkylene of 2 to 10 carbon atoms, or said alkylene interrupted by one to four -O-, -S- or -CH=CH-; or R₁₀ is arylene of 6 to 12 carbon atoms;
R₁₁ is alkylene of 2 to 20 carbon atoms or said alkylene interrupted by one to eight -O-; when n is 3, Q is a group -[(CH₂)ₘCOO]₃-R₁₂ where m is 1 to 3, and R₁₂ is an alkanetriyl of 3 to 12 carbon atoms;
when n is 4, Q is a group -[(CH₂)ₘCOO]₄-R₁₃ where m is 1 to 3, and R₁₃ is an alkanetetrayl of 4 to 12 carbon atoms;
Z₁ is a group of formula 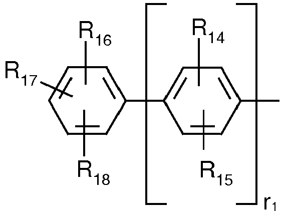
Z₂ is a group of formula 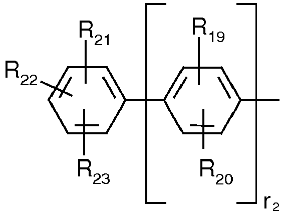 where
r₁ and r₂ are independently of each other 0 or 1;
R₁₄, R₁₅, R₁₆, R₁₇, R₁₈, R₁₉, R₂₀, R₂₁, R₂₂ and R₂₃ are independently of one another hydrogen, hydroxy, cyano, alkyl of 1 to 20 carbon atoms, alkoxy of 1 to 20 carbon atoms, phenylalkyl of 7 to 15 carbon atoms, cycloalkyl of 5 to 12 carbon atoms, cycloalkoxy of 5 to 12 carbon atoms, halogen, haloalkyl of 1 to 5 carbon atoms, sulfo, carboxy, acylamino of 2 to 12 carbon atoms, acyloxy of 2 to 12 carbon atoms, alkoxycarbonyl of 2 to 12 carbon atoms or aminocarbonyl; or R₁₇ and R₁₈ or R₂₂ and R₂₃ together with the phenyl radical to which they are attached are a cyclic radical interrupted by one to three -O- or -NR₅-.

For instance, alkyl or alkylene comprises at least 2 carbon atoms if said alkyl or said alkylene is interrupted by one or more groups (e.g. phenylene, -O-, -NR₅-, -CONR₅-, -COO-, -OCO-, -CO-, epoxy, -C(R₅)=C(R₅)COO-, -OCOC(R₅)=C(R₅)-, -C(R₅)=C(R₅)- or phenylene-G-phenylene groups).

When a denotation (e.g. R₂' or R₂") occurs more than once (e.g. twice) in a compound, this denotation may be different groups or the same group.

In the definitions the term alkyl comprises within the given limits of carbon atoms, for example methyl, ethyl, propyl, isopropyl, n-butyl, sec-butyl, isobutyl, tert-butyl, 2-ethylbutyl, n-pentyl, isopentyl, 1-methylpentyl, 1,3-dimethylbutyl, n-hexyl, 1-methylhexyl, n-heptyl, 2-methylheptyl, 1,1,3,3-tetramethylbutyl, 1-methylheptyl, 3-methylheptyl, n-octyl, 2-ethylhexyl, 1,1,3-trimethylhexyl, 1,1,3,3-tetramethylpentyl, nonyl, decyl, undecyl, 1-methylundecyl or dodecyl.

Examples of alkenyl are within the given limits of carbon atoms vinyl, allyl, 1-methylethenyl, and the branched and unbranched isomers of butenyl, pentenyl, hexenyl, heptenyl, octenyl, nonenyl, decenyl, undecenyl and dodecenyl. The term alkenyl also comprises residues with more than one double bond that may be conjugated or non-conjugated, for example may comprise one double bond.

Examples of alkylene are within the given limits of carbon atoms branched and unbranched isomers of ethylene, propylene, butylene, pentylene, hexylene, heptylene, octylene, nonylene, decylene, undecylene and dodecylene.

Some examples of cycloalkyl are cyclopentyl, cyclohexyl, methylcyclopentyl, dimethylcyclopentyl and methylcyclohexyl.

Some examples of cycloalkylene are cyclopentylene, cyclohexylene (e.g. 1,4-cyclohexylene), methylcyclopentylene, dimethylcyclopentylene and methylcyclohexylene.

Some examples of cycloalkenyl are cyclopentenyl, cyclohexenyl, methylcyclopentenyl, dimethylcyclopentenyl and methylcyclohexenyl. Cycloalkenyl may comprise more than one double bond that may be conjugated or non-conjugated, for example may comprise one double bond.

Aryl is for example phenyl.

Arylene is for instance phenylene, especially o-, m- or p-phenylene.

Examples of phenylene are o-, m- and p-phenylene.

Phenylalkyl is for instance benzyl or α,α-dimethylbenzyl.

The term alkoxy may comprise within the limits of the given number of carbon atoms, for example methoxy and ethoxy and the branched and unbranched isomers of propoxy, butoxy, pentyloxy, hexyloxy, heptyloxy and octyloxy.

In the definitions the term alkanoyl comprises within the given limits of carbon atoms, for example ethanoyl, propanoyl and branched and unbranched isomers of butanoyl, pentanoyl, hexanoyl, heptanoyl, octanoyl, nonanoyl, decanoyl, undecanoyl and dodecanoyl.

The term halogen may comprise chlorine, bromine and iodine; for example halogen is chlorine.

For example, a radical of a saturated or unsaturated bicyclic or tricyclic hydrocarbon of 7 to 12 carbon atoms is decaline.

Preferably, the 2-hydroxy phenyl triazine UV absorber (a) is of formula (I), (II) or (III), wherein X and Y are independently phenyl, or said phenyl, substituted by one to three alkyl of 1 to 6 carbon atoms, by hydroxy or by alkoxy of 1 to 6 carbon atoms or by mixtures thereof; or are independently Z₁ or Z₂;
R₁ is hydrogen;
R₁' and R₁" are as defined for R₁; R₂ is hydrogen, straight or branched chain alkyl of 1 to 24 carbon atoms; or said alkyl substituted by one to four -R₄, -OR₅, -N(R₅)₂, -CON(R₅)₂, -COR₅, -COOR₅, -OCOR₅, -OCOC(R₅)=C(R₅)₂, -C(R₅)=CCOOR₅, or combinations thereof; or said alkyl interrupted by one to four -O-, -NR₅-, -CONR₅-, -COO-, -OCO-, -CO-, -C(R₅)=C(R₅)COO-, -OCOC(R₅)=C(R₅)-, -C(R₅)=C(R₅)-, or combinations thereof, or said alkyl both substituted and interrupted by combinations of the groups mentioned above; or R₂ is -COR₆;
R₂, R₂' and R₂" are the same or different and are as defined for R₂;
R₄ is straight or branched chain alkenyl of 2 to 18 carbon atoms;
R₅ is defined as is R₄; or R₅ is also hydrogen or straight or branched chain alkyl of 1 to 24 carbon atoms, alkenyl of 2 to 24 carbon atoms;
R₆ is straight or branched chain alkyl of 1 to 18 carbon atoms, straight or branched chain alkenyl of 2 to 12 carbon atoms, alkylamino of 1 to 12 carbon atoms;
Z₁ is a group of formula 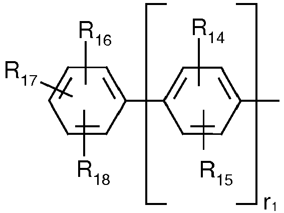 Z₂ is a group of formula 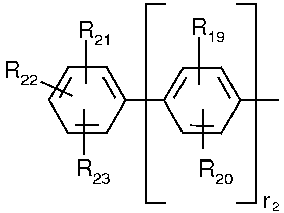 where
r₁ and r₂ are independently of each other 0 or 1;
R₁₄, R₁₅, R₁₆, R₁₇, R₁₈, R₁₉, R₂₀, R₂₁, R₂₂ and R₂₃ are independently of one another hydrogen, hydroxy, alkyl of 1 to 20 carbon atoms, alkoxy of 1 to 20 carbon atoms, carboxy, acylamino of 2 to 12 carbon atoms, acyloxy of 2 to 12 carbon atoms, alkoxycarbonyl of 2 to 12 carbon atoms or aminocarbonyl.

More preferably, the 2-hydroxy phenyl triazine UV absorber (a) is of formula (I), (II) or (III), especially of formula (I) or (III), wherein
X and Y are independently phenyl, or said phenyl, substituted by one to two alkyl of 1 carbon atom, by hydroxy or by alkoxy of 1 to 6 carbon atoms or by mixtures thereof; or are independently Z₁ or Z₂;
R₁ is hydrogen;
R₁' and R₁" are as defined for R₁;
R₂ is hydrogen, straight or branched chain alkyl of 1 to 15 carbon atoms; or said alkyl substituted by one or two -R₄, -OR₅, -COOR₅, -OCOR₅, -
OCOC(R₅)=C(R₅)₂, -C(R₅)=CCOOR₅, or combinations thereof; or said alkyl interrupted by one or two -O-, -COO-, -OCO- or combinations thereof, or said alkyl both substituted and interrupted by combinations of the groups mentioned above; or R₂ is -COR₆;
R₂, R₂' and R₂" are the same or different and are as defined for R₂;
R₄ is straight or branched chain alkenyl of 2 to 3 carbon atoms;
R₅ is defined as is R₄; or R₅ is also hydrogen or straight or branched chain alkyl of 1 to 15 carbon atoms, alkenyl of 2 to 3 carbon atoms;
R₆ is straight or branched chain alkyl of 1 to 15 carbon atoms, straight or branched chain alkenyl of 2 to 3 carbon atoms;
Z₁ is a group of formula 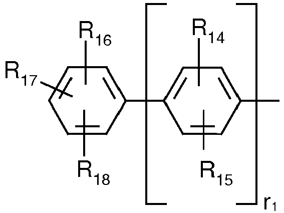 Z₂ is a group of formula 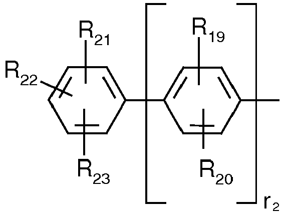 where
r₁ and r₂ are 1;
R₁₄, R₁₅, R₁₆, R₁₇, R₁₈, R₁₉, R₂₀, R₂₁, R₂₂ and R₂₃ are hydrogen.

Even more preferably, the 2-hydroxy phenyl triazine UV absorber (a) is of formula (I), (II) or (III), especially of formula (I) or (III), in particular of formula (I), wherein X and Y are independently phenyl, or said phenyl, substituted by one to two alkyl of 1 carbon atom, by hydroxy or by alkoxy of 1 to 6 carbon atoms or by mixtures thereof; or are independently Z₁ or Z₂;
R₁ is hydrogen;
R₁' and R₁" are as defined for R₁;
R₂ is hydrogen, straight or branched chain alkyl of 1 to 15 carbon atoms; or said alkyl substituted by one or two -OR₅, -COOR₅, -OCOR₅, -OCOC(R₅)=C(R₅)₂, or combinations thereof; or said alkyl interrupted by one -O-, -COO- or -OCO-, or said alkyl both substituted and interrupted by combinations of the groups mentioned above; or R₂ is -COR₆;
R₂, R₂' and R₂" are the same or different and are as defined for R₂;
R₅ is hydrogen or straight or branched chain alkyl of 1 to 15 carbon atoms, alkenyl of 2 to 3 carbon atoms;
R₆ is straight or branched chain alkenyl of 2 to 3 carbon atoms; Z₁ is a group of formula 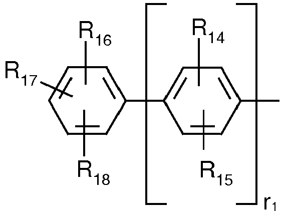 Z₂ is a group of formula 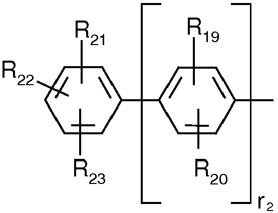 where
r₁ and r₂ are 1;
R₁₄, R₁₅, R₁₆, R₁₇, R₁₈, R₁₉, R₂₀, R₂₁, R₂₂ and R₂₃ are hydrogen.

For instance, the 2-hydroxy phenyl triazine UV absorber (a) is of formula (I), wherein X and Y are independently phenyl, or said phenyl, substituted by one to two alkyl of 1 carbon atom; or are independently Z₁ or Z₂;
R₁ is hydrogen;
R₂ is straight or branched chain alkyl of 1 to 15 carbon atoms; or said alkyl substituted by one -OR₅, -COOR₅ or -OCOR₅; or said alkyl interrupted by one -O-, -COO- or -OCO-, or said alkyl both substituted and interrupted by combinations of the groups mentioned above;
R₅ is hydrogen or straight or branched chain alkyl of 1 to 15 carbon atoms;
Z₁ and Z₂ are as defined above.

For instance, the 2-hydroxy phenyl triazine UV absorber (a) is of formula (I), wherein X and Y are phenyl substituted by two alkyl of 1 carbon atom; or are Z₁ or Z₂;
R₁ is hydrogen;
R₂ is straight or branched chain alkyl of 1 to 15 carbon atoms; or said alkyl substituted by one -OR₅ or -COOR₅; or said alkyl interrupted by one -O- or -COO-, or said alkyl both substituted and interrupted by combinations of the groups mentioned above;
R₅ is hydrogen or straight or branched chain alkyl of 1 to 15 carbon atoms; and Z₁ and Z₂ are as defined above.

Examples of suitable 2-hydroxy phenyl triazine UV absorbers are:
HPT-1 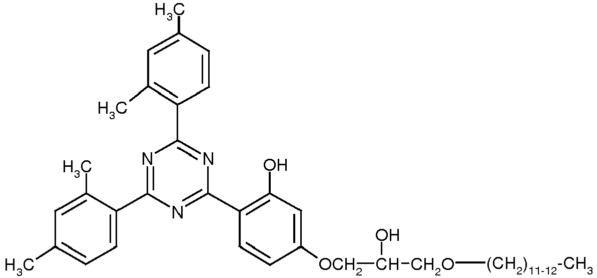
HPT-2 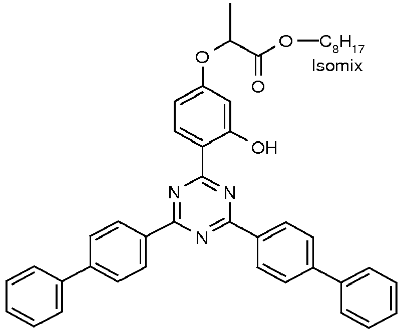 (example A14 of US6060543)
HPT-3 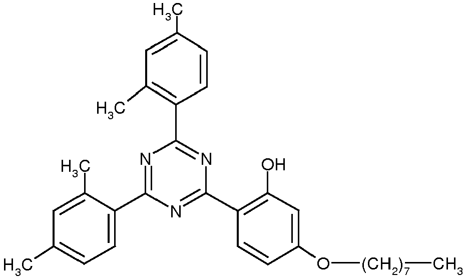
H PT-4 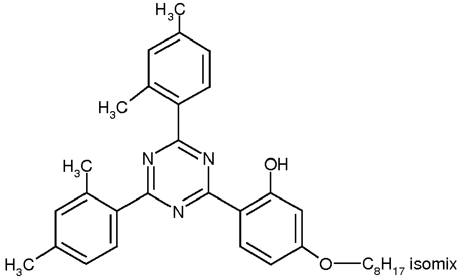
HPT-5 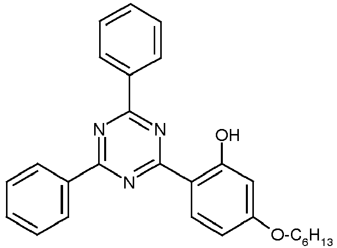
HPT-6 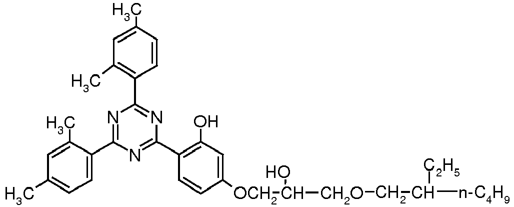
HPT-7 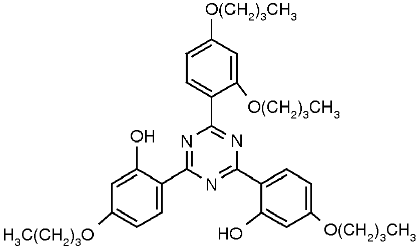
HPT-8 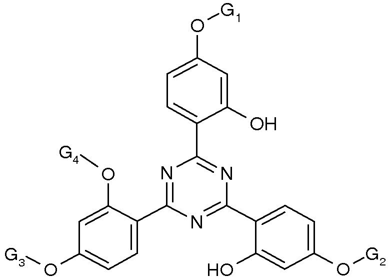 HPT-8 is a mixture of compounds with substituents as defined in 1), 2) and 3)
   1) G₁ = G₂ = CH(CH₃)-COO-C₈H₁₇, G₃ = G₄ = H;
   2) G₁ = G₂ = G₃ = CH(CH₃)-COO-C₈H₁₇, G₄ =H;
   3)G₁ = G₂ = G₃ = G₄ = CH(CH₃)-COO-C₈H₁₇;
H PT-9 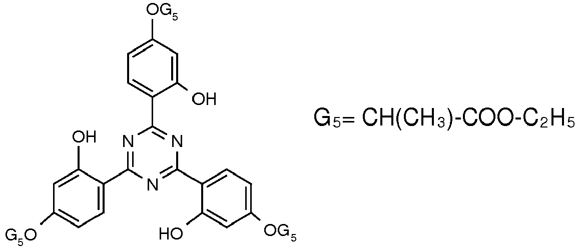
HPT-10 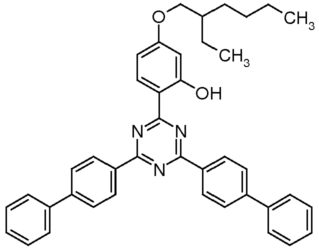 (example A8 of US6060543)
HPT-11 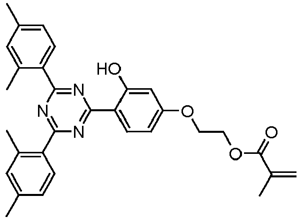
HPT-12 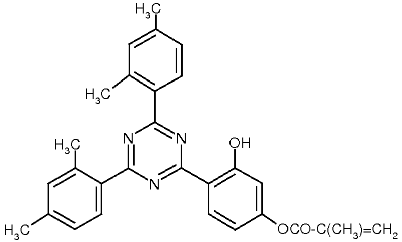
HPT-13 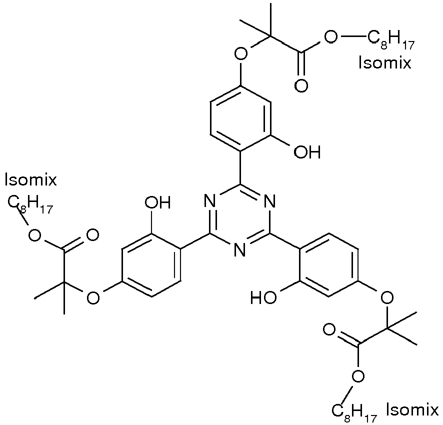
HPT-14 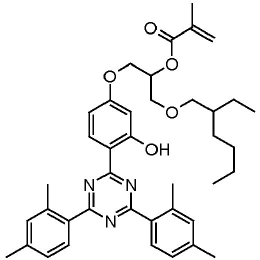
HPT-15 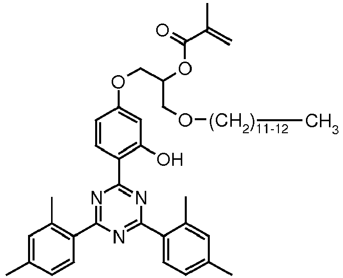
HPT-16 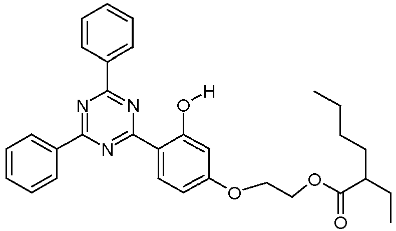
HPT-17 2,4,6-tris(2-hydroxy-4-octyloxyphenyl)-1,3,5-triazine,
HPT-18 2-(2,4-dihydroxyphenyl)-4,6-bis(2,4-dimethylphenyl)-1,3,5-triazine,
HPT-19 2,4-bis(2-hydroxy-4-propyloxyphenyl)-6-(2,4-dimethylphenyl)-1,3,5-triazine, HPT-20 2-(2-hydroxy-4-octyloxyphenyl)-4,6-bis(4-methylphenyl)-1,3,5-triazine,
H PT-21 2-(2-hydroxy-4-dodecyloxyphenyl)-4,6-bis(2,4-dimethylphenyl)-1,3,5-triazine,
HPT-22 2-(2-hydroxy-4-tridecyloxyphenyl)-4,6-bis(2,4-dimethylphenyl)-1,3,5-triazine,
HPT-23 2-[2-hydroxy-4-(2-hydroxy-3-butyloxypropoxy)phenyl]-4,6-bis(2,4-dimethyl)-1,3,5-triazine,
HPT-24 2-[2-hydroxy-4-(2-hydroxy-3-octyloxypropyloxy)phenyl]-4,6-bis(2,4-dimethyl)-1,3,5-triazine,
HPT-25 2-[2-hydroxy-4-(2-hydroxy-3-dodecyloxypropoxy)phenyl]-4,6-bis(2,4-dimethyl¬phenyl)-1,3,5-triazine,
HPT-26 2-(2-hydroxy-4-hexyloxy)phenyl-4,6-diphenyl-1,3,5-triazine,
HPT-27 2-(2-hydroxy-4-methoxyphenyl)-4,6-diphenyl-1,3,5-triazine,
HPT-28 2,4,6-tris[2-hydroxy-4-(3-butoxy-2-hydroxy¬propoxy)phenyl]-1,3,5-triazine,
HPT-29 2-(2-hydroxyphenyl)-4-(4-methoxyphenyl)-6-phenyl-1,3,5-tri¬azine,
HPT-30 2,4-bis(4-[2-ethylhexyloxy]-2-hydroxyphenyl)-6-(4-methoxyphe¬nyl)-1,3,5-triazine.

Most preferred 2-hydroxy phenyl triazine UV absorbers are HPT-1 to HPT-8, in particular HPT-1 to HPT-2.

A 2-hydroxy phenyl triazine UV absorber can be used per se or can be used as a concentrated aqueous polymer dispersion comprising the 2-hydroxy phenyl triazine UV absorber. Such concentrated aqueous polymer dispersions comprising 2-hydroxy phenyl triazine UV absorber can be for example as described in WO-A-05/023878 and can be obtained as described in WO-A-05/023878. The preferences for these concentrated aqueous polymer dispersions comprising 2-hydroxy phenyl triazine UV absorber can be the same as described herein for the product per se.

A further embodiment of this invention is component (a) which is a mixture of two or more different 2-hydroxy phenyl triazine UV absorbers.

For instance, component (a) is a mixture of two different 2-hydroxy phenyl triazine UV absorbers. Preferred mixtures are HPT-1 and HPT-2; HPT-2 and HPT-6; HPT-2 and HPT3; HPT-2 and HPT-4; HPT-2 and HPT-5; HPT-1 and HPT-7; HPT-1 and HPT-7; HPT-3 and HPT-7; HPT-4 and HPT-7; HPT-5 and HPT-7; HPT-6 and HPT-7; HPT-1 and HPT-8; HPT-3 and HPT-8; HPT-4 and HPT-8; HPT-5 and HPT-8; HPT-6 and HPT-8. Especially preferred are mixtures of HPT-1 and HPT-2. The ratio of the two different 2-hydroxy phenyl triazine UV absorbers in the mixture is, for instance, 4:1 to 1:4, preferably 3:1 to 1:3, even more preferred 2.5:1 to 1:2.5 by weight.

A rather specific example is for instance a mixture of HPT-1 and HPT-2 in a ratio of 4:1 to 1:4, preferably 4:1 to 1:1, even more preferred 2.5:1 to 1.5:1, most preferred about 2:1 by weight.

For example, component (a) is a mixture of three different 2-hydroxy phenyl triazine UV absorbers (e.g. HPT-1, HPT-2 and HPT-7; HPT-2, HPT-3 and HPT-7; HPT-2, HPT-4 and HPT-7; HPT-2, HPT-5 and HPT-7; HPT-2, HPT-6 and HPT-7; HPT-1, HPT-2 and HPT-8; HPT-2, HPT-3 and HPT-8; HPT-2, HPT-4 and HPT-8; HPT-2, HPT-5 and HPT-8; HPT-2, HPT-6 and HPT-8). The ratio of the first and second 2-hydroxy phenyl triazine UV absorber is for instance, 4:1 to 1:4, preferably 3:1 to 1:3, even more preferred 2.5:1 to 1:2.5. The ratio of the first and third 2-hydroxy phenyl triazine UV absorber is for instance, 4:1 to 1:4, preferably 3:1 to 1:3, even more preferably 2.5:1 to 1:2.5 by weight.

The 2-hydroxy phenyl triazine UV absorbers are partially commercially available. Otherwise they and their starting materials can be prepared by methods known in the art. For example, the 2-hydroxy phenyl triazine UV absorbers are prepared by Friedel-Crafts addition of halotriazines to corresponding aromatic compounds and phenols analogously to one of the methods specified in EP-A-434 608 or in one of the publications mentioned at the beginning or analogously to one of the methods specified in the publication by H. Brunetti and C.E. Lüthi, Helv. Chim. Acta 55, 1566 (1972); see also US Patent Nos. 5 726 310, 6 057 444, 6 225 468, and EP-A-941 989, WO 00/29392. That procedure can be followed by a further reaction according to known methods; such reactions and processes are described, for example, in EP-A-434 608. Hydroxyphenyl triazine UV absorber are described e.g. in GB-A-975966, EP-A-434608, US-4619956, US-5298067, EP-A-530135, EP-A-520938, EP-A-531258, US-5556973, US-5959008, US-6184375, US-6117997; for example in US-5998116, US-6255483, US-6060543.

The pigment (b) may be of any colour including black and white. The organic pigments may be those producing the colours commonly used in the pigment-using industries, such as the coating industry: namely black, blue, red, green, orange and yellow.

For further details as to organic pigments, reference is made to *Industrial Organic Pigments,* W. Herbst, K. Hunger, 2^{nd} edition, VCH Verlagsgesellschaft, Weinheim, 1997.

Particulary preferred pigments include C.I. Pigment Red 170, 177, 179, 202, 254, 264, C.I. Pigment Violet 19, 23, C.I. Pigment Blue 15, 15:1, 15:2, 15:3, 15:4, 15:6, 16, 60, C.I. Pigment Yellow 109, 110, 129, 138, 139, 150, 184, C.I. Pigment Green 7, 36, C.I. Pigment Orange 48, 73, a diketo-pyrrolo-pyrrol pigment, a quinacridone pigment, a quinacridone/diketo-pyrrolo-pyrrol pigment; as well as mixtures and solid solutions thereof.

The Colour Index (C.I.) is edited by the Society of Dyers and Colourists and the American Association of Textile Chemists and Colorists.

More particularly preferred pigments include C.I. Pigment Red 170, 177, 179, 202, 254, 264, C.I. Pigment Violet 19, 23, C.I. Pigment Blue 15, 15:1, 15:2, 15:3, 15:4, 15:6, 16, 60, C.I. Pigment Yellow 109, 110, 129, 138, 139, 150, 184, C.I. Pigment Green 7, 36, C.I. Pigment Orange 48, 73; as well as mixtures and solid solutions thereof.

Also more particularly preferred as pigment (b) is a quinacridone/diketo-pyrrolo-pyrrol pigment.

Such pigments are mainly commercially available. Otherwise such pigments can be prepared according to methods known in the art.

The binder (c) can in principle be any binder which is customary in industry, for example those described in Ullmann's Encyclopedia of Industrial Chemistry, 5th Edition, Vol. A18, pp. 368-426, VCH, Weinheim 1991. In general, it is a film forming binder based on a thermoplastic or thermosetting resin, predominantly on a thermosetting resin. Examples thereof are alkyd, acrylic, acrylic alkyd, polyester, phenolic, melamine, epoxy and polyurethane resins and mixtures thereof.

Component (c) can be a cold-curable or hot-curable binder; the addition of a curing catalyst may be advantageous. Suitable catalysts which accelerate curing of the binder are described, for example, in Ullmann's Encyclopedia of Industrial Chemistry, Vol. A18, p.469, VCH Verlagsgesellschaft, Weinheim 1991.

The present tinted clear coatings are for example employed as a top coating for automobiles.

Examples of coatings compositions containing specific binders are:
1. paints based on cold- or hot-crosslinkable alkyd, acrylate, polyester, epoxy or melamine resins or mixtures of such resins, if desired with addition of a curing catalyst;
2. two-component polyurethane paints based on hydroxyl-containing acrylate, polyester or polyether resins and aliphatic or aromatic isocyanates, isocyanurates or polyisocyanates;
3. one-component polyurethane paints based on blocked isocyanates, isocyanurates or polyisocyanates which are deblocked during baking, if desired with addition of a melamine resin;
4. one-component polyurethane paints based on a trisalkoxycarbonyltriazine crosslinker and a hydroxyl group containing resin such as acrylate, polyester or polyether resins;
5. one-component polyurethane paints based on aliphatic or aromatic urethaneacrylates or polyurethaneacrylates having free amino groups within the urethane structure and melamine resins or polyether resins, if necessary with curing catalyst;
6. two-component paints based on (poly)ketimines and aliphatic or aromatic isocyanates, isocyanurates or polyisocyanates;
7. two-component paints based on (poly)ketimines and an unsaturated acrylate resin or a polyacetoacetate resin or a methacrylamidoglycolate methyl ester;
8. two-component paints based on carboxyl- or amino-containing polyacrylates and polyepoxides;
9. two-component paints based on acrylate resins containing anhydride groups and on a polyhydroxy or polyamino component;
10. two-component paints based on acrylate-containing anhydrides and polyepoxides;
11. two-component paints based on (poly)oxazolines and acrylate resins containing anhydride groups, or unsaturated acrylate resins, or aliphatic or aromatic isocyanates, isocyanurates or polyisocyanates;
12. two-component paints based on unsaturated polyacrylates and polymalonates;
13. thermoplastic polyacrylate paints based on thermoplastic acrylate resins or externally crosslinking acrylate resins in combination with etherified melamine resins;
14. paint systems based on siloxane-modified or fluorine-modified acrylate resins.

For instance, component (c) is a binder for surface coatings, especially a binder for top coatings. Examples of such binders are an alkyd resin, a polyester resin, an acrylic resin, an epoxy resin, a polyurethane resin, a melamine/formaldehyde resin, a urea/formaldehyde resin, a blocked isocyanate resin and combinations thereof, especially an acrylic resin, a polyurethane resin, a blocked isocyanate resin and combinations thereof, in particular a polyurethane resin.

Possible drying catalysts or curing catalysts are, for example, organometallic compounds, amines, amino-containing resins and/or phosphines. Examples of organometallic compounds are metal carboxylates, especially those of the metals Pb, Mn, Co, Zn, Zr or Cu, or metal chelates, especially those of the metals Al, Ti or Zr, or organometallic compounds such as organotin compounds, for example.

Examples of metal carboxylates are the stearates of Pb, Mn or Zn, the octoates of Co, Zn or Cu, the naphthenates of Mn and Co or the corresponding linoleates, resinates or tallates.

Examples of metal chelates are the aluminium, titanium or zirconium chelates of acetylacetone, ethyl acetylacetate, salicylaldehyde, salicylaldoxime, o-hydroxyacetophenone or ethyl trifluoroacetylacetate, and the alkoxides of these metals.

Examples of organotin compounds are dibutyltin oxide, dibutyltin dilaurate or dibutyltin dioctoate.

Examples of amines are, in particular, tertiary amines, for example tributylamine, triethanolamine, N-methyldiethanolamine, N-dimethylethanolamine, N-ethylmorpholine, N-methylmorpholine or diazabicyclooctane (triethylenediamine) and salts thereof. Further examples are quaternary ammonium salts, for example trimethylbenzylammonium chloride.

Amino-containing resins are simultaneously binder and curing catalyst. Examples thereof are amino-containing acrylate copolymers.

The curing catalyst used can also be a phosphine, for example triphenylphosphine.

The coatings compositions can also be radiation-curable coating compositions. In this case, the binder essentially comprises monomeric or oligomeric compounds containing ethylenically unsaturated bonds, which after application are cured by actinic radiation, i.e. converted into a crosslinked, high molecular weight form. Where the system is UV-curing, it generally contains a photoinitiator as well. Corresponding systems are described in the abovementioned publication Ullmann's Encyclopedia of Industrial Chemistry, 5th Edition, Vol. A18, pages 451-453.

The coatings compositions according to the invention can be applied to any desired substrates, for example to metal, plastic or ceramic materials. They are for example used as a top coat in the finishing of automobiles.

The present coatings compositions may be applied to the substrates by the customary methods, for example by brushing, spraying, pouring, dipping or electrophoresis; see also Ullmann's Encyclopedia of Industrial Chemistry, 5th Edition, Vol. A18, pp. 491-500.

Depending on the binder system, the coatings may be cured at room temperature or by heating. The coatings may for example be cured at 50 - 150°C, and in the case of powder coatings or coil coatings even at higher temperatures.

The coatings compositions can comprise an organic solvent or solvent mixture in which the binder is soluble. The coatings compositions can otherwise be an aqueous solution or dispersion. The vehicle can also be a mixture of organic solvent and water. The coating composition may be a high-solids paint or can be solvent-free (e.g. a powder coating material). Powder coatings are, for example, those described in Ullmann's Encyclopedia of Industrial Chemistry, 5th Ed., A18, pages 438-444. The powder coating material may also have the form of a powder-slurry (dispersion of the powder preferably in water).

Preferably, the tinted clear coating composition as defined herein is an automotive coating composition.

Component (a) is typically present in an amount of from 0.2 to 20% by weight, preferably from 0.2 to 10% by weight, more preferably from 0.5 to 5% by weight, most preferably from 1.0 to 3.5%, by weight of the solid binder (c).

Component (b) is typically present in an amount of from 0.2 to 10% by weight, preferably from 0.2 to 5% by weight, more preferably from 0.4 to 3% by weight, most preferably from 0.5 to 1.5%, by weight of the solid binder (c).

For instance, the tinted clear coating composition comprises further additives.

Examples of such additives are:
1. Antioxidants
   1.1. Alkylated monophenols, for example 2,6-di-tert-butyl-4-methylphenol, 2-tert-butyl-4,6-dimethylphenol, 2,6-di-tert-butyl-4-ethylphenol, 2,6-di-tert-butyl-4-n-butylphenol, 2,6-di-tert-butyl-4-isobutylphenol, 2,6-dicyclopentyl-4-methylphenol, 2-(α-methylcyclohexyl)-4,6-dimethylphenol, 2,6-dioctadecyl-4-methylphenol, 2,4,6-tricyclohexylphenol, 2,6-di-tert-butyl-4-methoxymethylphenol, nonylphenols which are linear or branched in the side chains, for example, 2,6-di-nonyl-4-methylphenol, 2,4-dimethyl-6-(1'-methylundec-1'-yl)phenol, 2,4-dimethyl-6-(1'-methylheptadec-1'-yl)phenol, 2,4-dimethyl-6-(1'-methyltridec-1'-yl)phenol and mixtures thereof.
   1.2. Alkylthiomethylphenols, for example 2,4-dioctylthiomethyl-6-tert-butylphenol, 2,4-dioctyl-thiomethyl-6-methylphenol, 2,4-dioctylthiomethyl-6-ethylphenol, 2,6-di-dodecylthiomethyl-4-nonylphenol.
   1.3. Hydroquinones and alkylated hydroquinones, for example 2,6-di-tert-butyl-4-methoxyphenol, 2,5-di-tert-butylhydroquinone, 2,5-di-tert-amylhydroquinone, 2,6-diphenyl-4-octadecyloxyphenol, 2,6-di-tert-butylhydroquinone, 2,5-di-tert-butyl-4-hydroxyanisole, 3,5-di-tert-butyl-4-hydroxyanisole, 3,5-di-tert-butyl-4-hydroxyphenyl stearate, bis(3,5-di-tert-butyl-4-hydroxyphenyl) adipate.
   1.4. Tocopherols, for example α-tocopherol, β-tocopherol, γ-tocopherol, δ-tocopherol and mixtures thereof (vitamin E).
   1.5. Hydroxylated thiodiphenyl ethers, for example 2,2'-thiobis(6-tert-butyl-4-methylphenol), 2,2'-thiobis(4-octylphenol), 4,4'-thiobis(6-tert-butyl-3-methylphenol), 4,4'-thiobis(6-tert-butyl-2-methylphenol), 4,4'-thiobis(3,6-di-sec-amylphenol), 4,4'-bis(2,6-dimethyl-4-hydroxyphenyl)-disulfide.
   1.6. Alkylidenebisphenols, for example 2,2'-methylenebis(6-tert-butyl-4-methylphenol), 2,2'-methylenebis(6-tert-butyl-4-ethylphenol), 2,2'-methylenebis[4-methyl-6-(α-methylcyclohexyl)-phenol], 2,2'-methylenebis(4-methyl-6-cyclohexylphenol), 2,2'-methylenebis(6-nonyl-4-methylphenol), 2,2'-methylenebis(4,6-di-tert-butylphenol), 2,2'-ethylidenebis(4,6-di-tert-butylphenol), 2,2'-ethylidenebis(6-tert-butyl-4-isobutylphenol), 2,2'-methylenebis[6-(α-methylbenzyl)-4-nonylphenol], 2,2'-methylenebis[6-(α,α-dimethylbenzyl)-4-nonylphenol], 4,4'-methylenebis(2,6-di-tert-butylphenol), 4,4'-methylenebis(6-tert-butyl-2-methylphenol), 1,1-bis(5-tert-butyl-4-hydroxy-2-methylphenyl)butane, 2,6-bis(3-tert-butyl-5-methyl-2-hydroxybenzyl)-4-methylphenol, 1,1,3-tris(5-tert-butyl-4-hydroxy-2-methylphenyl)butane, 1,1-bis(5-tert-butyl-4-hydroxy-2-methyl-phenyl)-3-n-dodecylmercaptobutane, ethylene glycol bis[3,3-bis(3'-tert-butyl-4'-hydroxyphenyl)butyrate], bis(3-tert-butyl-4-hydroxy-5-methyl-phenyl)dicyclopentadiene, bis[2-(3'-tert-butyl-2'-hydroxy-5'-methylbenzyl)-6-tert-butyl-4-methylphenyl]terephthalate, 1,1-bis-(3,5-dimethyl-2-hydroxyphenyl)butane, 2,2-bis(3,5-di-tert-butyl-4-hydroxyphenyl)propane, 2,2-bis(5-tert-butyl-4-hydroxy2-methylphenyl)-4-n-dodecylmercaptobutane, 1,1,5,5-tetra-(5-tert-butyl-4-hydroxy-2-methylphenyl)pentane.
   1.7. O-, N- and S-benzyl compounds, for example 3,5,3',5'-tetra-tert-butyl-4,4'-dihydroxydibenzyl ether, octadecyl-4-hydroxy-3,5-dimethylbenzylmercaptoacetate, tridecyl-4-hydroxy-3,5-di-tert-butylbenzylmercaptoacetate, tris(3,5-di-tert-butyl-4-hydroxybenzyl)amine, bis(4-tert-butyl-3-hydroxy-2,6-dimethylbenzyl)dithioterephthalate, bis(3,5-di-tert-butyl-4-hydroxybenzyl)sulfide, isooctyl-3,5-di-tert-butyl-4-hydroxybenzylmercaptoacetate.
   1.8. Hydroxyenzylated malonates, for example dioctadecyl-2,2-bis(3,5-di-tert-butyl-2-hydroxybenzyl)malonate, di-octadecyl-2-(3-tert-butyl-4-hydroxy-5-methylbenzyl)malonate, di-dodecylmercaptoethyl-2,2-bis (3,5-di-tert-butyl-4-hydroxybenzyl)malonate, bis[4-(1,1,3,3-tetramethylbutyl)phenyl]-2,2-bis(3,5-di-tert-butyl-4-hydroxybenzyl)malonate.
   1.9. Aromatic hydroxybenzyl compounds, for example 1,3,5-tris(3,5-di-tert-butyl-4-hydroxybenzyl)-2,4,6-trimethylbenzene, 1,4-bis(3,5-di-tert-butyl-4-hydroxybenzyl)-2,3,5,6-tetramethylbenzene, 2,4,6-tris(3,5-di-tert-butyl-4-hydroxybenzyl)phenol.
   1.10. Triazine compounds, for example 2,4-bis(octylmercapto)-6-(3,5-di-tert-butyl-4-hydroxyanilino)-1,3,5-triazine, 2-octylmercapto-4,6-bis(3,5-di-tert-butyl-4-hydroxyanilino)-1,3,5-triazine, 2-octylmercapto-4,6-bis(3,5-di-tert-butyl-4-hydroxyphenoxy)-1,3,5-triazine, 2,4,6-tris-(3,5-di-tert-butyl-4-hydroxyphenoxy)-1,2,3-triazine, 1,3,5-tris(3,5-di-tert-butyl-4-hydroxybenzyl)isocyanurate, 1,3,5-tris(4-tert-butyl-3-hydroxy-2,6-dimethylbenzyl)isocyanurate, 2,4,6-tris-(3,5-di-tert-butyl-4-hydroxyphenylethyl)-1,3,5-triazine, 1,3,5-tris(3,5-di-tert-butyl-4-hydroxyphenylpropionyl)-hexahydro-1,3,5-triazine, 1,3,5-tris(3,5-dicyclohexyl-4-hydroxybenzyl)isocyanurate.
   1.11. Benzylphosphonates, for example dimethyl-2,5-di-tert-butyl-4-hydroxybenzylphosphonate, diethyl-3,5-di-tert-butyl-4-hydroxybenzylphosphonate, dioctadecyl3,5-di-tert-butyl-4-hydroxybenzylphosphonate, dioctadecyl-5-tert-butyl-4-hydroxy-3-methylbenzylphosphonate, the calcium salt of the monoethyl ester of 3,5-di-tert-butyl-4-hydroxybenzylphosphonic acid.
   1.12. Acylaminophenols, for example 4-hydroxylauranilide, 4-hydroxystearanilide, octyl N-(3,5-di-tert-butyl-4-hydroxyphenyl)carbamate.
   1.13. Esters of β-(3,5-di-tert-butyl-4-hydroxyphenyl)propionic acid with mono- or polyhydric alcohols, e.g. with methanol, ethanol, n-octanol, i-octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane.
   1.14. Esters of β-(5-tert-butyl-4-hydroxy-3-methylphenyl)propionic acid with mono- or polyhydric alcohols, e.g. with methanol, ethanol, n-octanol, i-octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis-(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane; 3,9-bis[2-{3-(3-tert-butyl-4-hydroxy-5-methylphenyl)propionyloxy}-1,1-dimethylethyl]-2,4,8,10-tetraoxaspiro[5.5]-undecane.
   1.15. Esters of β-(3,5-dicyclohexyl-4-hydroxyphenyl)propionic acid with mono- or polyhydric alcohols, e.g. with methanol, ethanol, octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane.
   1.16. Esters of 3,5-di-tert-butyl-4-hydroxvphenyl acetic acid with mono- or polyhydric alcohols, e.g. with methanol, ethanol, octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane.
   1.17. Amides of β-(3,5-di-tert-butyl-4-hydroxyphenyl)propionic acid e.g. N,N'-bis(3,5-di-tert-butyl-4-hydroxyphenylpropionyl)hexamethylenediamide, N,N'-bis(3,5-di-tert-butyl-4-hydroxyphenylpropionyl)trimethylenediamide, N,N'-bis(3,5-di-tert-butyl-4-hydroxyphenylpropionyl)hydrazide, N,N'-bis[2-(3-[3,5-di-tert-butyl-4-hydroxyphenyl]propionyloxy)ethyl]oxamide (Naugard®XL-1, supplied by Uniroyal).
   1.18. Ascorbic acid (vitamin C)
   1.19. Aminic antioxidants, for example N,N'-di-isopropyl-p-phenylenediamine, N,N'-di-sec-butyl-p-phenylenediamine, N,N'-bis(1,4-dimethylpentyl)-p-phenylenediamine, N,N'-bis(1-ethyl-3-methylpentyl)-p-phenylenediamine, N,N'-bis(1-methylheptyl)-p-phenylenediamine, N,N'-dicyclohexyl-p-phenylenediamine, N,N'-diphenyl-p-phenylenediamine, N,N'-bis(2-naphthyl)-p-phenylenediamine, N-isopropyl-N'-phenyl-p-phenylenediamine, N-(1,3-dimethylbutyl)-N'-phenyl-p-phenylenediamine, N-(1-methylheptyl)-N'-phenyl-p-phenylenediamine, N-cyclohexyl-N'-phenyl-p-phenylenediamine, 4-(p-toluenesulfamoyl)diphenylamine, N,N'-dimethyl-N,N'-di-sec-butyl-p-phenylenediamine, diphenylamine, N-allyldiphenylamine, 4-isopropoxydiphenylamine, N-phenyl-1-naphthylamine, N-(4-tert-octylphenyl)-1-naphthylamine, N-phenyl-2-naphthylamine, octylated diphenylamine, for example p,p'-di-tert-octyldiphenylamine, 4-n-butylaminophenol, 4-butyrylaminophenol, 4-nonanoylaminophenol, 4-dodecanoylaminophenol, 4-octadecanoylaminophenol, bis(4-methoxyphenyl)amine, 2,6-di-tert-butyl-4-dimethylamino-methylphenol, 2,4'-diaminodiphenylmethane, 4,4'-diaminodiphenylmethane, N,N,N',N'-tetramethyl-4,4'-diaminodiphenylmethane, 1,2-bis[(2-methylphenyl)amino]ethane, 1,2-bis(phenyl-amino)propane, (o-tolyl)biguanide, bis[4-(1',3'-dimethylbutyl)phenyl]amine, tert-octylated N-phenyl-1-naphthylamine, a mixture of mono- and dialkylated tert-butyl/tert-octyldiphenyl-amines, a mixture of mono- and dialkylated nonyldiphenylamines, a mixture of mono- and dialkylated dodecyldiphenylamines, a mixture of mono- and dialkylated isopropyl/isohexyl-diphenylamines, a mixture of mono- and dialkylated tert-butyldiphenylamines, 2,3-dihydro-3,3-dimethyl-4H-1,4-benzothiazine, phenothiazine, a mixture of mono- and dialkylated tert-butyl/tert-octylphenothiazines, a mixture of mono- and dialkylated tert-octyl-phenothiazines, N-allylphenothiazine, N,N,N',N'-tetraphenyl-1,4-diaminobut-2-ene.
2. UV absorbers and light stabilizers
   2.1. 2-(2'-Hydroxyphenyl)benzotriazoles, for example 2-(2'-hydroxy-5'-methylphenyl)-benzotriazole, 2-(3',5'-di-tert-butyl-2'-hydroxyphenyl)benzotriazole, 2-(5'-tert-butyl-2'-hydroxyphenyl)benzotriazole, 2-(2'-hydroxy-5'-(1,1,3,3-tetramethylbutyl)phenyl)benzotriazole, 2-(3',5'-di-tert-butyl-2'-hydroxyphenyl)-5-chloro-benzotriazole, 2-(3'-tert-butyl-2'-hydroxy-5'-methylphenyl)-5-chloro-benzotriazole, 2-(3'-sec-butyl-5'-tert-butyl-2'-hydroxyphenyl)benzotriazole, 2-(2'-hydroxy-4'-octyloxyphenyl)benzotriazole, 2-(3',5'-di-tert-amyl-2'-hydroxyphenyl)benzotriazole, 2-(3',5'-bis-(α,α-dimethylbenzyl)-2'-hydroxyphenyl)benzotriazole, 2-(3'-tert-butyl-2'-hydroxy-5'-(2-octyloxycarbonylethyl)phenyl)-5-chloro-benzotriazole, 2-(3'-tert-butyl-5'-[2-(2-ethylhexyloxy)-carbonylethyl]-2'-hydroxyphenyl)-5-chloro-benzotriazole, 2-(3'-tert-butyl-2'-hydroxy-5'-(2-methoxycarbonylethyl)phenyl)-5-chloro-benzotriazole, 2-(3'-tert-butyl-2'-hydroxy-5'-(2-methoxycarbonylethyl)phenyl)benzotriazole, 2-(3'-tert-butyl-2'-hydroxy-5'-(2-octyloxycarbonylethyl)phenyl)benzotriazole, 2-(3'-tert-butyl-5'-[2-(2-ethylhexyloxy)carbonylethyl]-2'-hydroxyphenyl)benzotriazole, 2-(3'-dodecyl-2'-hydroxy-5'-methylphenyl)benzotriazole, 2-(3'-tert-butyl-2'-hydroxy-5'-(2-isooctyloxycarbonylethyl)phenylbenzotriazole, 2,2'-methylene-bis[4-(1,1,3,3-tetramethylbutyl)-6-benzotriazole-2-ylphenol]; the transesterification product of 2-[3'-tert-butyl-5'-(2-methoxycarbonylethyl)-2'-hydroxyphenyl]-2H-benzotriazole with polyethylene glycol 300; 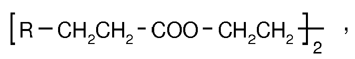 where R = 3'-tert-butyl-4'-hydroxy-5'-2H-benzotriazol-2-ylphenyl, 2-[2'-hydroxy-3'-(α,α-dimethylbenzyl)-5'-(1,1,3,3-tetramethylbutyl)-phenyl]-benzotriazole; 2-[2'-hydroxy-3'-(1,1,3,3-tetramethylbutyl)-5'-(α,α-dimethylbenzyl)-phenyl]benzotriazole.
   2.2. 2-Hydroxybenzophenones, for example the 4-hydroxy, 4-methoxy, 4-octyloxy, 4-decyloxy, 4-dodecyloxy, 4-benzyloxy, 4,2',4'-trihydroxy and 2'-hydroxy-4,4'-dimethoxy derivatives.
   2.3. Benzoates, such as esters of substituted and unsubstituted benzoic acids, for example 4-tert-butyl-phenyl salicylate, phenyl salicylate, octylphenyl salicylate, dibenzoyl resorcinol, bis(4-tert-butylbenzoyl)resorcinol, benzoyl resorcinol, 2,4-di-tert-butylphenyl 3,5-di-tert-butyl-4-hydroxybenzoate, hexadecyl 3,5-di-tert-butyl-4-hydroxybenzoate, octadecyl 3,5-di-tert-butyl-4-hydroxybenzoate, 2-methyl-4,6-di-tert-butylphenyl 3,5-di-tert-butyl-4-hydroxybenzoate.
   2.4. Acrylates, for example ethyl α-cyano-β,β-diphenylacrylate, isooctyl α-cyano-β,β-diphenylacrylate, methyl α-carbomethoxycinnamate, methyl α-cyano-β-methyl-p-methoxycinnamate, butyl α-cyano-β-methyl-p-methoxy-cinnamate, methyl α-carbomethoxy-p-methoxycinnamate, N-(β-carbomethoxy-β-cyanovinyl)-2-methylindoline, neopentyl tetra(α-cyano-β,β-diphenylacrylate.
   2.5. Nickel compounds, for example nickel complexes of 2,2'-thio-bis[4-(1,1,3,3-tetramethylbutyl)phenol], such as the 1:1 or 1:2 complex, with or without additional ligands such as n-butylamine, triethanolamine or N-cyclohexyldiethanolamine, nickel dibutyldithiocarbamate, nickel salts of the monoalkyl esters, e.g. the methyl or ethyl ester, of 4-hydroxy-3,5-di-tert-butylbenzylphosphonic acid, nickel complexes of ketoximes, e.g. of 2-hydroxy-4-methylphenylundecylketoxime, nickel complexes of 1-phenyl-4-lauroyl-5-hydroxypyrazole, with or without additional ligands.
   2.6. Sterically hindered amine stabilizers, for example bis(2,2,6,6-tetramethyl-4-piperidyl)sebacate, bis(2,2,6,6-tetramethyl-4-piperidyl)succinate, bis(1,2,2,6,6-pentamethyl-4-piperidyl)sebacate, bis(1-octyloxy-2,2,6,6-tetramethyl-4-piperidyl)sebacate, bis(1,2,2,6,6-pentamethyl-4-piperidyl) n-butyl-3,5-di-tert-butyl-4-hydroxybenzylmalonate, the condensate of 1-(2-hydroxyethyl)-2,2,6,6-tetramethyl-4-hydroxypiperidine and succinic acid, linear or cyclic condensates of N,N'-bis(2,2,6,6-tetramethyl-4-piperidyl)hexamethylenediamine and 4-tert-octylamino-2,6-dichloro-1,3,5-triazine, tris(2,2,6,6-tetramethyl-4-piperidyl)nitrilotriacetate, tetrakis(2,2,6,6-tetramethyl-4-piperidyl)-1,2,3,4-butanetetracarboxylate, 1,1'-(1,2-ethanediyl)-bis(3,3,5,5-tetramethylpiperazinone), 4-benzoyl-2,2,6,6-tetramethylpiperidine, 4-stearyloxy-2,2,6,6-tetramethylpiperidine, bis(1,2,2,6,6-pentamethylpiperidyl)-2-n-butyl-2-(2-hydroxy-3,5-di-tert-butylbenzyl)malonate, 3-n-octyl-7,7,9,9-tetramethyl-1,3,8-triazaspiro[4.5]decane-2,4-dione, bis(1-octyloxy-2,2,6,6-tetramethylpiperidyl)sebacate, bis(1-octyloxy-2,2,6,6-tetramethylpiperidyl)succinate, linear or cyclic condensates of N,N'-bis(2,2,6,6-tetramethyl-4-piperidyl)hexamethylenediamine and 4-morpholino-2,6-dichloro-1,3,5-triazine, the condensate of 2-chloro-4,6-bis(4-n-butylamino-2,2,6,6-tetramethylpiperidyl)-1,3,5-triazine and 1,2-bis(3-aminopropylamino)ethane, the condensate of 2-chloro-4,6-di-(4-n-butylamino-1,2,2,6,6-pentamethylpiperidyl)-1,3,5-triazine and 1,2-bis(3-aminopropylamino)ethane, 8-acetyl-3-dodecyl-7,7,9,9-tetramethyl-1,3,8-triazaspiro[4.5]decane-2,4-dione, 3-dodecyl-1-(2,2,6,6-tetramethyl-4-piperidyl)pyrrolidine-2,5-dione, 3-dodecyl-1-(1,2,2,6,6-pentamethyl-4-piperidyl)pyrrolidine-2,5-dione, a mixture of 4-hexadecyloxy- and 4-stearyloxy-2,2,6,6-tetramethylpiperidine, a condensate of N,N'-bis(2,2,6,6-tetramethyl-4-piperidyl)hexamethylenediamine and 4-cyclohexylamino-2,6-dichloro-1,3,5-triazine, a condensate of 1,2-bis(3-aminopropylamino)ethane and 2,4,6-trichloro-1,3,5-triazine as well as 4-butylamino-2,2,6,6-tetramethylpiperidine (CAS Reg. No. [136504-96-6]); a condensate of 1,6-hexanediamine and 2,4,6-trichloro-1,3,5-triazine as well as N,N-dibutylamine and 4-butylamino-2,2,6,6-tetramethylpiperidine (CAS Reg. No. [192268-64-7]); N-(2,2,6,6-tetramethyl-4-piperidyl)-n-dodecylsuccinimide, N-(1,2,2,6,6-pentamethyl-4-piperidyl)-n-dodecylsuccinimide, 2-undecyl-7,7,9,9-tetramethyl-1-oxa-3,8-diaza-4-oxo-spiro[4,5]decane, a reaction product of 7,7,9,9-tetramethyl-2-cycloundecyl-1-oxa-3,8-diaza-4-oxospiro-[4,5]decane and epichlorohydrin, 1,1-bis(1,2,2,6,6-pentamethyl-4-piperidyloxycarbonyl)-2-(4-methoxyphenyl)ethene, N,N'-bis-formyl-N,N'-bis(2,2,6,6-tetramethyl-4-piperidyl)hexamethylenediamine, a diester of 4-methoxymethylenemalonic acid with 1,2,2,6,6-pentamethyl-4-hydroxypiperidine, poly[methylpropyl-3-oxy-4-(2,2,6,6-tetramethyl-4-piperidyl)]siloxane, a reaction product of maleic acid anhydride-α-olefin copolymer with 2,2,6,6-tetramethyl-4-aminopiperidine or 1,2,2,6,6-pentamethyl-4-aminopiperidine, 2,4-bis[N-(1-cyclohexyloxy-2,2,6,6-tetramethylpiperidine-4-yl)-N-butylamino]-6-(2-hydroxyethyl)amino-1,3,5-triazine, 1-(2-hydroxy-2-methylpropoxy)-4-octadecanoyloxy-2,2,6,6-tetramethylpiperidine, 5-(2-ethylhexanoyl)-oxymethyl-3,3,5-trimethyl-2-morpholinone, Sanduvor (Clariant; CAS Reg. No. 106917-31-1], 5-(2-ethylhexanoyl)oxymethyl-3,3,5-trimethyl-2-morpholinone, the reaction product of 2,4-bis-[(1-cyclohexyloxy-2,2,6,6-piperidine-4-yl)butylamino]-6-chloro-s-triazine with N,N'-bis(3-aminopropyl)ethylenediamine), 1,3,5-tris(N-cyclohexyl-N-(2,2,6,6-tetramethylpiperazine-3-one-4-yl)amino)-s-triazine, 1,3,5-tris(N-cyclohexyl-N-(1,2,2,6,6-pentamethylpiperazine-3-one-4-yl)-amino)-s-triazine.
   2.7. Oxamides, for example 4,4'-dioctyloxyoxanilide, 2,2'-diethoxyoxanilide, 2,2'-dioctyloxy-5,5'-di-tert-butoxanilide, 2,2'-didodecyloxy-5,5'-di-tert-butoxanilide, 2-ethoxy-2'-ethyloxanilide, N,N'-bis(3-dimethylaminopropyl)oxamide, 2-ethoxy-5-tert-butyl-2'-ethoxanilide and its mixture with 2-ethoxy-2'-ethyl-5,4'-di-tert-butoxanilide, mixtures of o- and p-methoxy-disubstituted oxanilides and mixtures of o- and p-ethoxy-disubstituted oxanilides.
3. Metal deactivators, for example N,N'-diphenyloxamide, N-salicylal-N'-salicyloyl hydrazine, N,N'-bis(salicyloyl)hydrazine, N,N'-bis(3,5-di-tert-butyl-4-hydroxyphenylpropionyl)hydrazine, 3-salicyloylamino-1,2,4-triazole, bis(benzylidene)oxalyl dihydrazide, oxanilide, isophthaloyl dihydrazide, sebacoyl bisphenylhydrazide, N,N'-diacetyladipoyl dihydrazide, N,N'-bis(salicyloyl)oxalyl dihydrazide, N,N'-bis(salicyloyl)thiopropionyl dihydrazide.
4. Phosphites and phosphonites, for example triphenyl phosphite, diphenylalkyl phosphites, phenyldialkyl phosphites, tris(nonylphenyl) phosphite, trilauryl phosphite, trioctadecyl phosphite, distearylpentaerythritol diphosphite, tris(2,4-di-tert-butylphenyl) phosphite, diisodecyl pentaerythritol diphosphite, bis(2,4-di-tert-butylphenyl)pentaerythritol diphosphite, bis(2,4-di-cumylphenyl)pentaerythritol diphosphite, bis(2,6-di-tert-butyl-4-methylphenyl)pentaerythritol diphosphite, diisodecyloxypentaerythritol diphosphite, bis(2,4-di-tert-butyl-6-methylphenyl)-pentaerythritol diphosphite, bis(2,4,6-tris(tert-butylphenyl)pentaerythritol diphosphite, tristearyl sorbitol triphosphite, tetrakis(2,4-di-tert-butylphenyl) 4,4'-biphenylene diphosphonite, 6-isooctyloxy-2,4,8,10-tetra-tert-butyl-12H-dibenz[d,g]-1,3,2-dioxaphosphocin, bis(2,4-di-tert-butyl-6-methylphenyl)methyl phosphite, bis(2,4-di-tert-butyl-6-methylphenyl)ethyl phosphite, 6-fluoro-2,4,8,10-tetra-tert-butyl-12-methyl-dibenz[d,g]-1,3,2-dioxaphosphocin, 2,2',2"-nitrilo-[triethyltris(3,3',5,5'-tetra-tert-butyl-1,1'-biphenyl-2,2'-diyl)phosphite], 2-ethylhexyl(3,3',5,5'-te-tra-tert-butyl-1,1'-biphenyl-2,2'-diyl)phosphite, 5-butyl-5-ethyl-2-(2,4,6-tri-tert-butylphenoxy)-1,3,2-dioxaphosphirane.

The following phosphites are especially preferred:
Tris(2,4-di-tert-butylphenyl) phosphite (Irgafos®168, Ciba Specialty Chemicals Inc.), tris(nonylphenyl) phosphite,

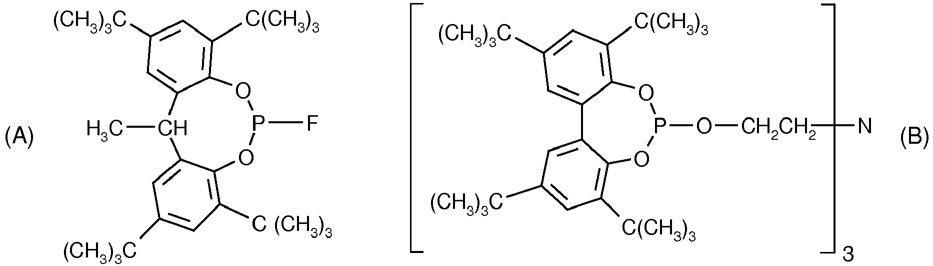

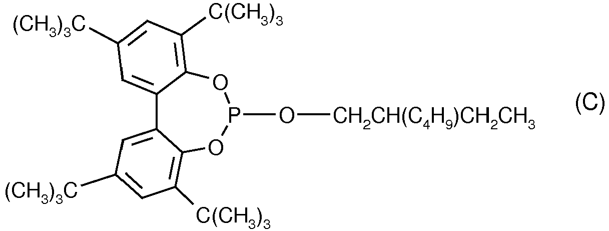

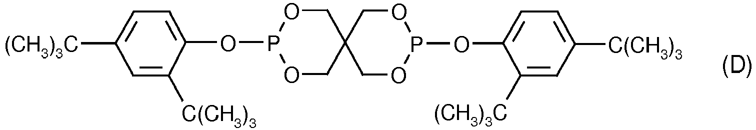

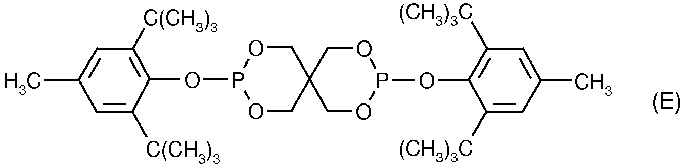

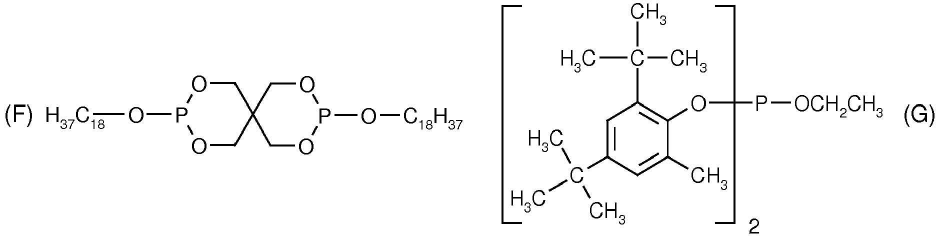

5. Hydroxylamines, for example N,N-dibenzylhydroxylamine, N,N-diethylhydroxylamine, N,N-dioctylhydroxylamine, N,N-dilaurylhydroxylamine, N,N-ditetradecylhydroxylamine, N,N-dihexadecylhydroxylamine, N,N-dioctadecylhydroxylamine, N-hexadecyl-N-octadecylhydroxylamine, N-heptadecyl-N-octadecylhydroxylamine, N,N-dialkylhydroxylamine derived from hydrogenated tallow amine.
6. Nitrones, for example, N-benzyl-alpha-phenylnitrone, N-ethyl-alpha-methylnitrone, N-octyl-alpha-heptylnitrone, N-lauryl-alpha-undecylnitrone, N-tetradecyl-alpha-tridecylnnitrone, N-hexadecyl-alpha-pentadecylnitrone, N-octadecyl-alpha-heptadecylnitrone, N-hexadecyl-alpha-heptadecylnitrone, N-ocatadecyl-alpha-pentadecylnitrone, N-heptadecyl-alpha-heptadecylnitrone, N-octadecyl-alpha-hexadecylnitrone, nitrone derived from N,N-dialkylhydroxylamine derived from hydrogenated tallow amine.
7. Thiosynergists, for example dilauryl thiodipropionate, dimistryl thiodipropionate, distearyl thiodipropionate or distearyl disulfide.
8. Peroxide scavengers, for example esters of β-thiodipropionic acid, for example the lauryl, stearyl, myristyl or tridecyl esters, mercaptobenzimidazole or the zinc salt of 2-mercaptobenzimidazole, zinc dibutyldithiocarbamate, dioctadecyl disulfide, pentaerythritol tetrakis(β-dodecylmercapto)propionate.
9. Polyamide stabilizers, for example copper salts in combination with iodides and/or phosphorus compounds and salts of divalent manganese.
10. Basic co-stabilizers, for example melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids, for example calcium stearate, zinc stearate, magnesium behenate, magnesium stearate, sodium ricinoleate and potassium palmitate, antimony pyrocatecholate or zinc pyrocatecholate.
11. Nucleating agents, for example inorganic substances, such as talcum, metal oxides, such as titanium dioxide or magnesium oxide, phosphates, carbonates or sulfates of, preferably, alkaline earth metals; organic compounds, such as mono- or polycarboxylic acids and the salts thereof, e.g. 4-tert-butylbenzoic acid, adipic acid, diphenylacetic acid, sodium succinate or sodium benzoate; polymeric compounds, such as ionic copolymers (ionomers). Especially preferred are 1,3:2,4-bis(3',4'-dimethylbenzylidene)sorbitol, 1,3:2,4-di(paramethyl-dibenzylidene)sorbitol, and 1,3:2,4-di(benzylidene)sorbitol.
12. Fillers and reinforcing agents, for example calcium carbonate, silicates, glass fibres, glass beads, asbestos, talc, kaolin, mica, barium sulfate, metal oxides and hydroxides, carbon black, graphite, flours or fibers of natural products, synthetic fibers.
13. Other additives, for example plasticisers, lubricants, emulsifiers, rheology additives, catalysts, flow-control agents, optical brighteners, flameproofing agents, antistatic agents and blowing agents.
14. Benzofuranones and indolinones, for example those disclosed in U.S. 4,325,863; U.S. 4,338,244; U.S. 5,175,312; U.S. 5,216,052; U.S. 5,252,643; DE-A-4316611; DE-A-4316622; DE-A-4316876; EP-A-0589839, EP-A-0591102; EP-A-1291384 or 3-[4-(2-acetoxyethoxy)phenyl]-5,7-di-tert-butylbenzofuran-2-one, 5,7-di-tert-butyl-3-[4-(2-stearoyloxy-ethoxy)phenyl]benzofuran-2-one, 3,3'-bis[5,7-di-tert-butyl-3-(4-[2-hydroxyethoxy]phenyl)benzofuran-2-one], 5,7-di-tert-butyl-3-(4-ethoxyphenyl)benzofuran-2-one, 3-(4-acetoxy-3,5-dimethylphenyl)-5,7-di-tert-butylbenzofuran-2-one, 3-(3,5-dimethyl-4-pivaloyloxyphenyl)-5,7-di-tert-butylbenzofuran-2-one, 3-(3,4-dimethylphenyl)-5,7-di-tert-butylbenzofuran-2-one, 3-(2,3-dimethylphenyl)-5,7-di-tert-butylbenzofuran-2-one, 3-(2-acetyl-5-isooctylphenyl)-5-isooctyl-benzofuran-2-one.

These further additives are generally used in an amount of from 0.01 to 25% by weight, especially from 0.1 to 10% by weight, in particular from 0.5 to 5% by weight, of the solid binder (c).

Preferred are further additives selected from the group consisting of phenolic and aminic antioxidants, sterically hindered amine stabilizers, UV-absorbers different from those as defined herein as 2-hydroxyphenyl triazines, phosphites, phosphonites, benzofuranones, metal stearates, metal oxides, organophosphorus compounds, hydroxylamines, flame retardants and mixtures thereof.

More preferred are further additives selected from the group consisting of sterically hindered amine stabilizers and UV absorbers. The UV-absorbers are preferably selected from the group consisting of the oxamides, the 2-hydroxybenzophenones, the benzoates, the acrylates and the 2-(2'-hydroxyphenyl) benzotriazoles. For instance, the oxamides are as listed as item 2.7, the 2-hydroxybenzophenones as item 2.2, the benzoates as item 2.3, the acrylates as item 2.4 and the 2-(2'-hydroxyphenyl) benzotriazoles as item 2.1 in the list above.

Particularly preferred as additives are sterically hindered amine stabilizers such as listed as item 2.6 in the list above and mixtures thereof. Especially preferred is bis(1-octyloxy-2,2,6,6-tetramethyl-4-piperidyl)sebacate. Further examples of sterically hindered amine stabilizers are as listed in GB-A-2347928 from page 4, line 6 to page 27, penultimate paragraph, WO-A-01/92392 from page 1, line 8 to page 49, penultimate paragraph and in WO-A-03/076505 from page 10, paragraph 6 to page 39, paragraph 5 and can be prepared as described therein.

The sterically hindered amine stabilizers are present from 0.01 to 10 % by weight, preferably from 0.1 to 5% by weight, more preferably from 0.5 to 3% by weight, most preferably from 0.5 to 2% by weight, of the solid binder (c).

Typically, the additives (e.g. pigments, 2-hydroxyphenyl triazines, sterically hindered amine stabilizers) are dispersed in a dispersion of binder and solvent and then added to the coating composition or the additives are dispersed directly in the coating composition.

A further embodiment of the invention is a tinted clear coating obtained by applying a tinted clear coating composition as defined herein on a substrate.

Preferably, the tinted clear coating is the top coating. For instance, the substrate is another coating (which is denoted herein as "substrate coating"), metal, plastic or ceramic materials.

For example, the substrate coating is applied on another coating, metal, plastic or ceramic materials, especially another coating or metal. Preferably, the substrate coating comprises at least one binder and at least one pigment; for instance, the ratio of pigment to solid binder is from 1:1 to 1:8 by weight, preferably from 1:2 to 1:6 by weight. Typically, the substrate coating does not contain UV-absorbers and sterically hindered amine stabilizers. The substrate coating is usually opaque. The binder of the substrate coating is for instance as described above for binder (c) and can be a mixture of binders.

The pigment is selected from the group consisting of quinacridone, diketo-pyrrolo-pyrrole, quinacridone/diketo-pyrrolo-pyrrole, isoindolinone, phthalocyanine, perylene, anthraquinone Red, indanthrone Blue, azomethine Cu complex, mono azo Ni complex, quinophthalone, isoindoline and naphthol AS. The pigment may be a solid solution pigment. Mixtures of the pigments may also be used. Mixtures of crystal combinations of pigments may be used. For further details as to all those organic pigments, reference is made to Industrial Organic Pigments, W. Herbst, K. Hunger, 2nd edition, VCH Verlagsgesellschaft, Weinheim, 1997.

Such pigments are mainly commercially available. Otherwise such pigments can be prepared according to methods known in the art.

For instance, if the substrate is a substrate coating, the pigment(s) (b) of the tinted clear coating are highly transparent or semi opaque pigment(s) or mixtures thereof and the pigment(s) of the substrate coating are opaque pigment(s).

For example, the pigment or mixture of pigments (b) of the tinted clear coating is of a similar color shade as the pigment of mixture of pigments of the substrate coating.

Preferably, the tinted clear coating is an automotive coating.

More preferably, the tinted clear coating is an automotive coating comprising the following layers
(d) a cathodically deposited coating, adhering to a metal substrate;
(e) at least one subsequent coating layer adhering to the cathodically deposited coating;
(f) at least one base coating layer containing a pigment or mixtures of pigments; and
(g) a tinted clear coating as defined herein.
For instance, the coating layer (e) is directly next to the coating layer (d), the coating layer (f) is directly next to the coating layer (e) and the coating layer (g) is directly next to the coating layer (f).

The base coating layer (f) is typically as defined above for the substrate coating. The preferences for layer (f) and (g) are typically as defined above for a tinted clear coating on a substrate coating.

For example, in such an automotive coating, the metal substrate is pre-treated in e.g. a customary zinc phosphate bath.

A further embodiment of the invention is a process for the preparation of a tinted clear coating which comprises the applying of a tinted clear coating composition as defined herein to a substrate.

For instance, the substrate is an automobile. For example the tinted clear coating is an automotive coating.

Preferred is a process, wherein the substrate comprises the following layers
(d) a cathodically deposited coating, adhering to a metal substrate;
(e) at least one subsequent coating layer adhering to the cathodically deposited coating; and
(f) at least one base coating layer containing a pigment or mixtures of pigments.

The preferences for these processes are as outlined above for the tinted clear coating and tinted clear coating composition.

All % are weight-% unless otherwise stated. All ratios are weight ratios unless otherwise stated.

### Abbreviations:

- ": inch
- C.I.: color index
- DFT: dry film thickness
- P/B: pigment to binder ratio

Abbreviation for products used in the application examples: 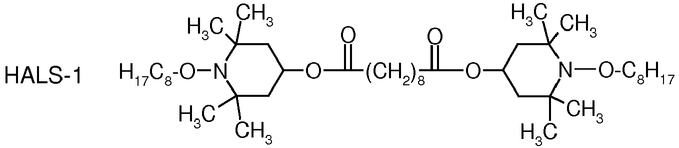 P-1 CINQUASIA® BRILLIANT RED RT-380-D (a solid solution pigment based on quinacridone and diketo-pyrrolo-pyrrole; supplier: Ciba® Specialty Chemicals)

### Application Examples: Comparison of a tinted clear coating against an untinted clear coating

### Example 1

A pigmented base coating formulation (see below) is sprayed on a backing (Q-Panel Type: Special QTY 125: Coil coated Aluminium 0.025"x4"x12") and dried 30 min in the air. The resulting dry film thickness of the pigmented base coating is approximately 16µm.

A tinted clear coating formulation (according to invention) or an untinted clear coating formulation (comparison; i.e. a clear coating) is sprayed over the pigmented base coating and dried 30 min in the air and 30 min in a stove at 130°C. The resulting dry film thickness of the tinted or untinted clear coating is 35 µm - 40 µm.

### Pigmented base coating formulation

**Table 1: Base coating formulation:**

| | Initial weight (in delivery form) | Solid content % | Supplier |
|---|---|---|---|
| Dejon. water | 38.00g | | |
| Maprenal MF 900¹⁾ | 0.54g | 0.50 | Ineos |
| Surfynol 104E²⁾ | 1.50g | 0.80 | AIR PRODUCTS |
| EnviroGem® AEO2³⁾ | 0.50g | 0.50 | AIR PRODUCTS |
| Proglyde DMM⁴⁾ | 0.80g | | DOW |
| DOWANOL Pnp⁵⁾ | 4.50g | | DOW |
| DOWANOL DPnP⁶⁾ | 0.80g | | DOW |
| n-Butanol | 1.50g | | |
| Ciba® VISCALEX®⁷⁾ HV 30 (1:1 water) | 5.30g | 0.80 | Ciba Specialty Chemicals |
| Dimethylethanolamin (10% in water) | 3.20g | | |
| ALBERDINGK® APU 10120 VP⁸⁾ | 43.16g | 18.60 | ALBERDINGK-BOLEY |
| ACTICIDE MBS⁹⁾ | 0.20g | | Thor Gmbh |

| | | | |
|---|---|---|---|
| Raw Materials: ¹⁾Maprenal MF 900: Melamine resin HMMM ²⁾ Surfynol 104E: Acetylenic diol, Antifoaming / wetting agent ³⁾ EnviroGem AE02: Tenside, Antifoaming / wetting agent ⁴⁾ Proglyde DMM: Dipropylen-Glycol-Dimethyl-Ether, Aprotic coalescing agent ⁵⁾ DOWANOL PnP: Propylene Glycol-n-Propyl Ether, Fast evaporation coupling agent ⁶⁾ DOWANOL DPnP: Dipropylene Glycol n-Propyl Ether, Evaporation Coalescing or coupling agent ⁷⁾ VISCALEX HV 30: Acrylic copolymer emulsion in water, thickening and rheology agent ⁸⁾ APU 10120 VP Acrylic-polyurethane copolymer, Binder ⁹⁾ ACTICIDE MBS: Metyl-4-lsothiacolin, Biocide | | | |

**Table 2: mill bases of pigments for the base coating**

| Pigment | %* Pigment | %* solid binder |
|---|---|---|
| C.I. Pigment Red 202 | 24.00 | 13.20 |
| C.I. Pigment Red 179 | 17.00 | 39.90 |
| Aluminium | 22.46 | 34.54 |
| C.I. Pigment Black 7 | 6.00 | 16.50 |

| | | |
|---|---|---|
| * % by weight of mill base, separately for each pigment | | |

**Table 3: ratio of pigment mixture for the base coating**

| red metallic pigment mixture | Ratio |
|---|---|
| C.I. Pigment Red 202 | 26.40 |
| C.I. Pigment Red 179 | 33.43 |
| Aluminium | 39.56 |
| C.I. Pigment Black 7 | 0.61 |

### Preparation of the pigmented base coating formulation:

Each pigment is dispersed individually on a horizontal bead mill. The dispersed Mill bases are blended together and are added to the base coating formulation. The pigment to solid binder ratio is adjusted to 1/3.6.

### Tinted and untinted clear coating formulations

**Table 4: 2K PU clear coating formulation**

| | Initial weight |
|---|---|
| Macrynal SM 510n (60% supply form)¹⁾ | 75.0g |
| Butylgycol acetate | 15.0g |
| Solvesso 100²⁾ | 6.1 g |
| Methyl isobutyl ketone | 3.6g |
| Zn octoate catalyst (8% metal) | 0.1g |
| BYK 300³⁾ | 0.2g |
| | |
| Total polyol component | 100.0g |

Pigment preparation of the mill base formulation for the tinted clear coating with the following P/B ratio:

| Pigment | % Pigment of mill base formulation | % solid binder of mill base formulation |
|---|---|---|
| P-1 | 15.00 | 19.77 |

The light stabilizers (see Table 5 below; HALS and HPT) are added to the above polyol component, where they readily dissolve.

For tinted clear coatings, the pigment (P) is dispersed on a horizontal bead mill with solid binder and is added to the above polyol component by incorporation of the above described mill base under agitation.

Prior to application 40g Desmodur N 75⁴⁾ (isocyanate component) are added to the above polyol component. The resulting clear coating formulation (solids content: 50%) is subsequently sprayed onto the above base coating resulting after cure (130°C, 30') in a dry film thickness of 35-40µm.

**Raw Materials:**

| | |
|---|---|
| ¹⁾ Macrynal SM 510n: | acrylic polyol resin (ca. 4.5% hydroxyl content based on solid resin); |
| Supplier: | Solutia (formerly Vianova Resins) |
| ²⁾ Solvesso 100: | aromatic hydrocarbon; supplier: Exxon |
| ³⁾ BYK 300: | levelling agent; supplier: BYK - Chemie |
| ⁴⁾ Desmodur N 75: | isocyanate component; supplier: Bayer AG |

The panels are coloristically evaluated and subsequently exposed for 4000h according to SAE-J 1960 (Xe-WOM exposure). The color measurements are conducted on a X-Rite SP68 Spectrophotometer, specular included and parameter calculation (according to DIN 6174) is performed with CGREC software.

**Table 5: Chroma and durability comparison of tinted and untinted clear coating panels**

| Additives (% on solid binder) | | Kind of clear coating | Chroma C* (initial) | DE (after 4000h exposure) |
|---|---|---|---|---|
| HPT-1 | 2.4% | untinted | 32.50 | 1.1 |
| HALS-1 | 1.0% | | | |
| P-1 | 1.0% | tinted | 39.00 | 0.6 |
| HPT-1 | 2.4% | | | |
| HALS-1 | 1.0% | | | |

Tinted clear coating panel shows a significantly increased Chroma compared to untinted clear coating panel as well as better durability, i.e. less colour deviation.

### Example 2

The tinted and untinted clear coating panels are produced as in Example 1 except that the stabilizers of the tinted and untinted clear coatings are modified (for details see Table 6).

**Table 6: Chroma and durability comparison of tinted and untinted clear coating panels**

| Additives (% on solid binder) | | Kind of clear coating | Chroma C* (initial) | DE (after 4000h exposure) |
|---|---|---|---|---|
| HPT-1 | 1.8% | untinted | 32.50 | 0.9 |
| HPT-2 | 0.9% | | | |
| HALS-1 | 1.0% | | | |
| P-1 | 1.0% | tinted | 39.00 | 6 |
| HPT-1 | 1.8% | | | |
| HPT-2 | 0.9% | | | |
| HALS-1 | 1.0% | | | |

Tinted clear coating panel shows a significantly increased Chroma compared to untinted clear coating panel as well as better durability, i.e. less colour deviation.

## Claims

1. A tinted clear coating composition comprising
(a) at least one 2-hydroxy phenyl triazine UV absorber,
(b) at least one pigment selected from the group consisting of quinacridone, diketo-pyrrolo-pyrrole, quinacridone/diketo-pyrrolo-pyrrole, isoindolinone, phthalocyanine, perylene, anthraquinone Red, indanthrone Blue, azomethine Cu complex, mono azo Ni complex, quinophthalone, isoindoline and naphthol AS and mixtures and solid solutions thereof, wherein the ratio of component (a) to component (b) is from 10:1 to 1:1 by weight, and
(c) at least one binder.

2. A tinted clear coating compositions according to claim 1, wherein the 2-hydroxy phenyl triazine UV absorber is of formula (I), (II), (III), (IV), (V) or (VI) 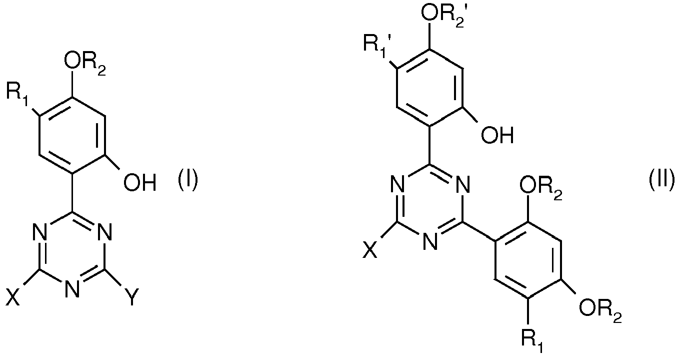 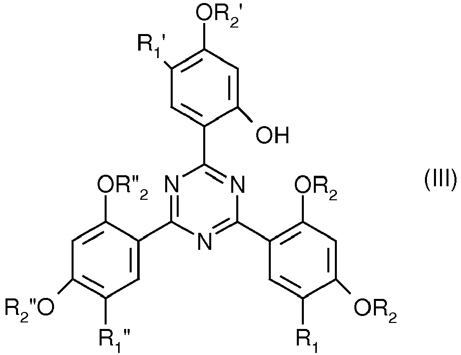 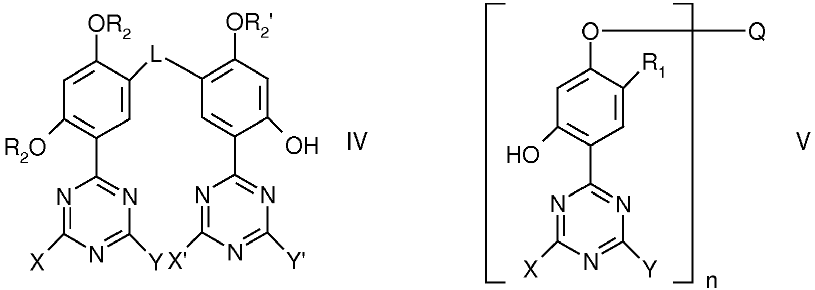 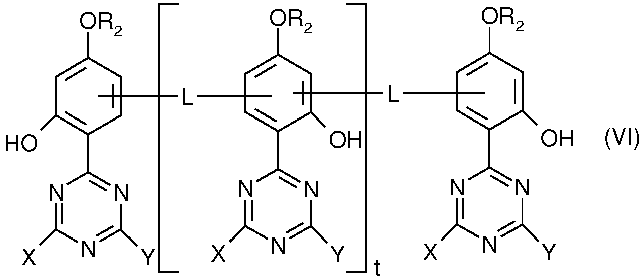 wherein
X and Y are independently phenyl, naphthyl, pyrenyl, phenanthrenyl or fluoranthenyl, or said phenyl, said naphthyl, said pyrenyl, said phenanthrenyl or said fluoranthenyl substituted by one to three alkyl of 1 to 6 carbon atoms, by halogen, by hydroxy or by alkoxy of 1 to 6 carbon atoms or by mixtures thereof; or are independently Z₁ or Z₂;
X, X', Y and Y' are the same or different and are as defined for X and Y;
R₁ is hydrogen, straight or branched chain alkyl of 1 to 24 carbon atoms, cycloalkyl of 5 to 12 carbon atoms, phenylalkyl of 7 to 15 carbon atoms, halogen, -SR₃, -SOR₃ or -SO₂R₃; or said alkyl, said cycloalkyl or said phenylalkyl substituted by one to three halogen, -R₄, -OR₅, -N(R₅)₂, -COR₅, -COOR₅, -OCOR₅, -CN, -NO₂, -SR₅, -SOR₅, -SO₂R₅ or -P(O)(OR₅)₂, morpholinyl, piperidinyl, 2,2,6,6-tetramethylpiperidinyl, piperazinyl or N-methylpiperidinyl groups or combinations thereof; or said alkyl or said cycloalkyl interrupted by one to four phenylene, -O-, -NR₅-, -CONR₅-, -COO-, -OCO- or -CO groups or combinations thereof; or said alkyl or said cycloalkyl both substituted and interrupted by combinations of the groups mentioned above;
R₁, R₁' and R₁" are the same or different and are as defined for R₁ ;
R₂ is hydrogen, straight or branched chain alkyl of 1 to 24 carbon atoms or cycloalkyl of 5 to 12 carbon atoms; or said alkyl or said cycloalkyl substituted by one to four halogen, epoxy, glycidyloxy, furyloxy, -R₄, -OR₅, -N(R₅)₂, -CON(R₅)₂, -COR₅, -COOR₅, -OCOR₅, -OCOC(R₅)=C(R₅)₂, -C(R₅)=CCOOR₅, -CN, -NCO, or 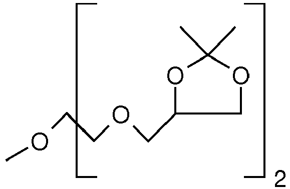 or combinations thereof; or said alkyl or said cycloalkyl interrupted by one to four epoxy, -O-, -NR₅-, -CONR₅-, -COO-, -OCO-, -CO-, -C(R₅)=C(R₅)COO-, -OCOC(R₅)=C(R₅)-, - C(R₅)=C(R₅)-, phenylene or phenylene-G-phenylene in which G is -O-, -S-, -SO₂-, -CH₂- or - C(CH₃)₂- or combinations thereof, or said alkyl or said cycloalkyl both substituted and interrupted by combinations of the groups mentioned above; or R₂ is -SO₂R₃ or -COR₆;
R₂, R₂' and R₂" are the same or different and are as defined for R₂;
R₃ is alkyl of 1 to 20 carbon atoms, alkenyl of 3 to 18 carbon atoms, cycloalkyl of 5 to 12 carbon atoms, phenylalkyl of 7 to 15 carbon atoms, aryl of 6 to 10 carbon atoms or said aryl substituted by one or two alkyl of 1 to 4 carbon atoms;
R₄ is aryl of 6 to 10 carbon atoms or said aryl substituted by one to three halogen, alkyl of 1 to 8 carbon atoms, alkoxy of 1 to 8 carbon atoms or combinations thereof; cycloalkyl of 5 to 12 carbon atoms; phenylalkyl of 7 to 15 carbon atoms or said phenylalkyl substituted on the phenyl ring by one to three halogen, alkyl of 1 to 8 carbon atoms, alkoxy of 1 to 8 carbon atoms or combinations thereof; or straight or branched chain alkenyl of 2 to 18 carbon atoms;
R₅ is defined as is R₄; or R₅ is also hydrogen or straight or branched chain alkyl of 1 to 24 carbon atoms, alkenyl of 2 to 24 carbon atoms; or R₅ is a group for formula 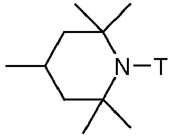
T is hydrogen, oxyl, hydroxyl, -OT₁, alkyl of 1 to 24 carbon atoms, said alkyl substituted by one to three hydroxy; benzyl or alkanoyl of 2 to 18 carbon atoms;
T₁ is alkyl of 1 to 24 carbon atoms, cycloalkyl of 5 to 12 carbon atoms, alkenyl of 2 to 24 carbon atoms, cycloalkenyl of 5 to 12 carbon atoms, phenylalkyl of 7 to 15 carbon atoms, a radical of a saturated or unsaturated bicyclic or tricyclic hydrocarbon of 7 to 12 carbon atoms or aryl of 6 o 10 carbon atoms or said aryl substituted by one to three alkyl of 1 to 4 carbon atoms;
R₆ is straight or branched chain alkyl of 1 to 18 carbon atoms, straight or branched chain alkenyl of 2 to 12 carbon atoms, phenoxy, alkylamino of 1 to 12 carbon atoms, arylamino of 6 to 12 carbon atoms, -R₇COOH or -NH-R₈-NCO;
R₇ is alkylene of 2 to 14 carbon atoms or phenylene;
R₈ is alkylene of 2 to 24 carbon atoms, phenylene, tolylene, diphenylmethane or a group 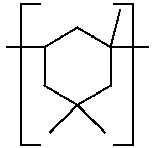
t is 0 to 9;
L is straight or branched alkylene of 1 to 12 carbon atoms, cycloalkylene of 5 to 12 carbon atoms or alkylene substituted or interrupted by cyclohexylene or phenylene; or L is benzylidene; or L is -S-, -S-S-, -S-E-S-, -SO-, -SO₂-, -SO-E-SO-, -SO₂-E-SO₂-, -CH₂-NH-E-NH-CH₂- or 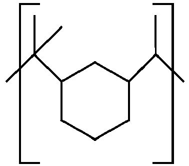
E is alkylene of 2 to 12 carbon atoms, cycloalkylene of 5 to 12 carbon atoms or alkylene interrupted or terminated by cycloalkylene of 5 to 12 carbon atoms;
n is 2, 3 or 4;
when n is 2; Q is straight or branched alkylene of 2 to 16 carbon atoms; or said alkylene substituted by one to three hydroxy groups; or said alkylene interrupted by one to three -CH=CH- or -O-; or said alkylene both substituted and interrupted by combinations of the groups mentioned above; or Q is xylylene or a group -CONH-R₈-NHCO-, - CH₂CH(OH)CH₂O-R₉-OCH₂CH(OH)CH₂-, -CO-R₁₀-CO-, or -(CH₂)ₘ-COO-R₁₁-OOC-(CH₂)ₘ-, where m is 1 to 3; or Q is 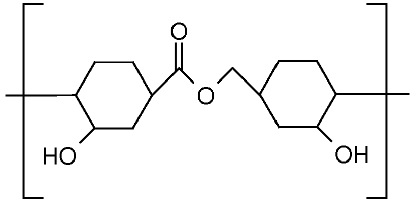
R₉ is alkylene of 2 to 50 carbon atoms; or said alkylene interrupted by one to ten -O-, phenylene or a group -phenylene-G-phenylene in which G is -O-, -S-, -SO₂-, -CH₂- or -C(CH₃)₂-;
R₁₀ is alkylene of 2 to 10 carbon atoms, or said alkylene interrupted by one to four -O-, -S- or -CH=CH-; or R₁₀ is arylene of 6 to 12 carbon atoms;
R₁₁ is alkylene of 2 to 20 carbon atoms or said alkylene interrupted by one to eight -O-; when n is 3, Q is a group -[(CH₂)ₘCOO]₃-R₁₂ where m is 1 to 3, and R₁₂ is an alkanetriyl of 3 to 12 carbon atoms;
when n is 4, Q is a group -[(CH₂)ₘCOO]₄-R₁₃ where m is 1 to 3, and R₁₃ is an alkanetetrayl of 4 to 12 carbon atoms;
Z₁ is a group of formula 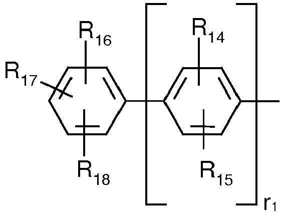
Z₂ is a group of formula 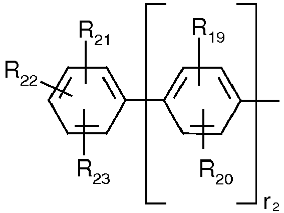 where
r₁ and r₂ are independently of each other 0 or 1;
R₁₄, R₁₅, R₁₆, R₁₇, R₁₈, R₁₉, R₂₀, R₂₁, R₂₂ and R₂₃ are independently of one another hydrogen, hydroxy, cyano, alkyl of 1 to 20 carbon atoms, alkoxy of 1 to 20 carbon atoms, phenylalkyl of 7 to 15 carbon atoms, cycloalkyl of 5 to 12 carbon atoms, cycloalkoxy of 5 to 12 carbon atoms, halogen, haloalkyl of 1 to 5 carbon atoms, sulfo, carboxy, acylamino of 2 to 12 carbon atoms, acyloxy of 2 to 12 carbon atoms, alkoxycarbonyl of 2 to 12 carbon atoms or aminocarbonyl; or R₁₇ and R₁₈ or R₂₂ and R₂₃ together with the phenyl radical to which they are attached are a cyclic radical interrupted by one to three -O- or -NR₅-.

3. A tinted clear coating composition according to claim 2, wherein component (a) is a 2-hydroxy phenyl triazine UV absorber of formula (I), (II) or (III), wherein
X and Y are independently phenyl, or said phenyl, substituted by one to three alkyl of 1 to 6 carbon atoms, by hydroxy or by alkoxy of 1 to 6 carbon atoms or by mixtures thereof; or are independently Z₁ or Z₂;
R₁ is hydrogen;
R₁' and R₁" are as defined for R₁;
R₂ is hydrogen, straight or branched chain alkyl of 1 to 24 carbon atoms; or said alkyl substituted by one to four -R₄, -OR₅, -N(R₅)₂, -CON(R₅)₂, -COR₅, -COOR₅, -OCOR₅, -OCOC(R₅)=C(R₅)₂, -C(R₅)=CCOOR₅, or combinations thereof; or said alkyl interrupted by one to four -O-, -NR₅-, -CONR₅-, -COO-, -OCO-, -CO-, -C(R₅)=C(R₅)COO-, -OCOC(R₅)=C(R₅)-, -C(R₅)=C(R₅)-, or combinations thereof, or said alkyl both substituted and interrupted by combinations of the groups mentioned above; or R₂ is -COR₆;
R₂, R₂' and R₂" are the same or different and are as defined for R₂;
R₄ is straight or branched chain alkenyl of 2 to 18 carbon atoms;
R₅ is defined as is R₄; or R₅ is also hydrogen or straight or branched chain alkyl of 1 to 24 carbon atoms, alkenyl of 2 to 24 carbon atoms;
R₆ is straight or branched chain alkyl of 1 to 18 carbon atoms, straight or branched chain alkenyl of 2 to 12 carbon atoms, alkylamino of 1 to 12 carbon atoms;
Z₁ is a group of formula 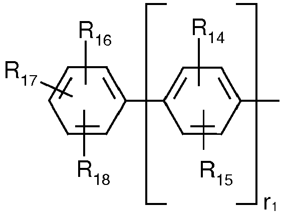 Z₂ is a group of formula 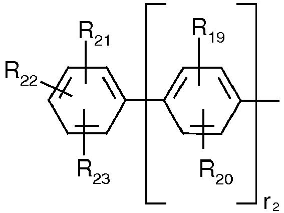 where
r₁ and r₂ are independently of each other 0 or 1;
R₁₄, R₁₅, R₁₆, R₁₇, R₁₈, R₁₉, R₂₀, R₂₁, R₂₂ and R₂₃ are independently of one another hydrogen, hydroxy, alkyl of 1 to 20 carbon atoms, alkoxy of 1 to 20 carbon atoms, carboxy, acylamino of 2 to 12 carbon atoms, acyloxy of 2 to 12 carbon atoms, alkoxycarbonyl of 2 to 12 carbon atoms or aminocarbonyl.

4. A tinted clear coating composition according to any one of claims 1-3, wherein component (a) is a mixture of two or more different 2-hydroxy phenyl triazine UV absorber.

5. A tinted clear coating composition according to claim 1-4, wherein component (b) is a C.I. Pigment Red 170, 177, 179, 202, 254, 264, a C.I. Pigment Violet 19, 23, a C.I. Pigment Blue 15, 15:1, 15:2, 15:3, 15:4, 15:6, 16, 60, a C.I. Pigment Yellow 109, 110, 129, 138, 139, 150, 184, a C.I. Pigment Green 7, 36, a C.I. Pigment Orange 48, 73, a diketo-pyrrolo-pyrrol pigment, a quinacridone pigment, a quinacridone/diketo-pyrrolo-pyrrol pigment; as well as mixtures or solid solutions thereof.

6. A tinted clear coating composition according to any one of claims 1-5, wherein component (c) is an alkyd resin, a polyester resin, an acrylic resin, an epoxy resin, a polyurethane resin, a melamine/formaldehyde resin, a urea/formaldehyde resin, a blocked isocyanate resin or combinations thereof.

7. A tinted clear coating composition according to any one of claims 1-6, wherein the coating composition is an automotive coating composition.

8. A tinted clear coating composition according to any one of claims 1-7, wherein component (a) is present in an amount of from 0.2 to 20% by weight of the solid binder (c).

9. A tinted clear coating composition according to any one of claims 1-8, wherein component (b) is present in an amount of from 0.2 to 10% by weight of the solid binder (c).

10. A tinted clear coating composition according to any one of claims 1-9, which comprises further additives.

11. A tinted clear coating composition according to claim 10, which comprises as further additives phenolic and/or aminic antioxidants, sterically hindered amine stabilizers, UV-absorbers different from those as defined in claim 1, phosphites, phosphonites, benzofuranones, metal stearates, metal oxides, organophosphorus compounds, hydroxylamines and/or flame retardants.

12. A tinted clear coating composition according to claim 11, which comprises as further additives sterically hindered amine stabilizers and/or UV absorbers selected from the group consisting of the oxamides, the 2-hydroxybenzophenones, the benzoates, the acrylates and the 2-(2'-hydroxyphenyl) benzotriazoles.

13. A tinted clear coating obtained by applying a tinted clear coating composition of any one of claims 1-12 on a substrate.

14. A tinted clear coating according to claim 13 which is an automotive coating.

15. A tinted clear coating according to claim 14, wherein the automotive coating comprises the following layers
(d) a cathodically deposited coating, adhering to a metal substrate;
(e) at least one subsequent coating layer adhering to the cathodically deposited coating;
(f) at least one base coating layer containing a pigment or mixtures of pigments;
(g) a tinted clear coating as defined in claim 13.

16. A process for the preparation of a tinted clear coating which comprises the applying of a tinted clear coating composition as of any one of claims 1-12 to a substrate.

17. A process according to claim 16, wherein the substrate comprises the following layers
(d) a cathodically deposited coating, adhering to a metal substrate;
(e) at least one subsequent coating layer adhering to the cathodically deposited coating; and
(f) at least one base coating layer containing a pigment or mixtures of pigments.

## Patentansprüche

1. Getönte Klarlackzusammensetzung, umfassend
(a) mindestens einen 2-Hydroxyphenyltriazin-UV-Absorber,
(b) mindestens ein Pigment aus der Gruppe bestehend aus Chinacridon, Diketopyrrolopyrrol, Chinacridon/Diketopyrrolopyrrol, Isoindolinon, Phthalocyanin, Perylen, Anthrachinonrot, Indanthronblau, Azomethin-Cu-Komplex, Monoazo-Ni-Komplex, Chinophthalon, Isoindolin und Naphthol AS und Mischungen und Mischkristallen davon, wobei das Gewichtsverhältnis von Komponente (a) zu Komponente (b) 10:1 bis 1:1 beträgt, und
(c) mindestens ein Bindemittel.

2. Getönte Klarlackzusammensetzung nach Anspruch 1, wobei der 2-Hydroxyphenyltriazin-UV-Absorber die Formel (I), (II), (III), (IV), (V) oder (VI) aufweist: 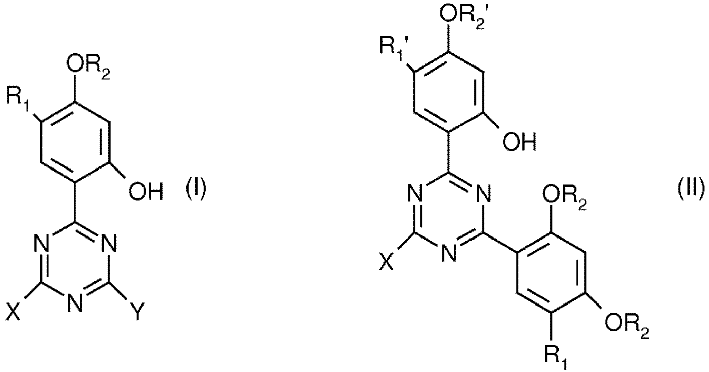 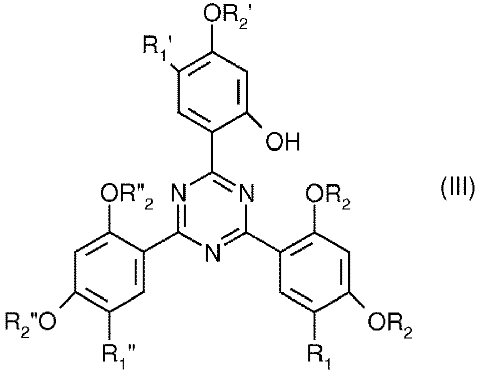 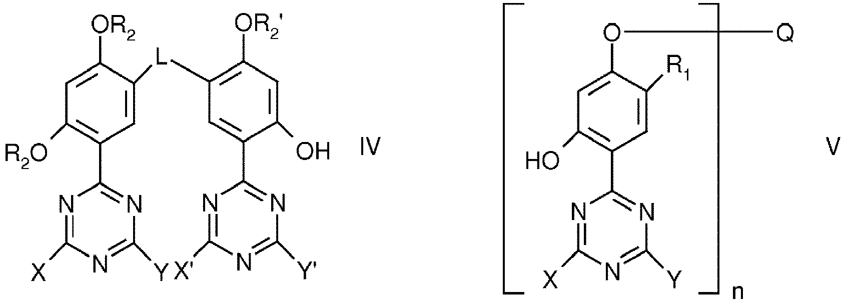 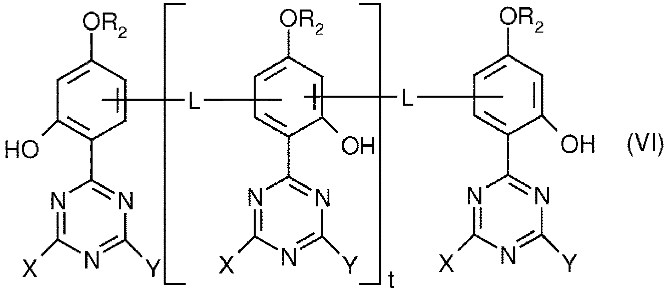 wobei
X und Y unabhängig für Phenyl, Naphthyl, Pyrenyl, Phenanthrenyl oder Fluoranthenyl oder das Phenyl, das Naphthyl, das Pyrenyl, das Phenanthrenyl oder das Fluoranthenyl, das durch ein bis drei Alkyl mit 1 bis 6 Kohlenstoffatomen, durch Halogen, durch Hydroxy oder durch Alkoxy mit 1 bis 6 Kohlenstoffatomen oder durch Mischungen davon substituiert ist, oder unabhängig für Z₁ oder Z₂ stehen;
X, X', Y und Y' gleich oder verschieden sind und wie für X und Y definiert sind;
R₁ für Wasserstoff, gerad- oder verzweigtkettiges Alkyl mit 1 bis 24 Kohlenstoffatomen, Cycloalkyl mit 5 bis 12 Kohlenstoffatomen, Phenylalkyl mit 7 bis 15 Kohlenstoffatomen, Halogen, -SR₃, -SOR₃ oder -SO₂R₃ oder das Alkyl, das Cycloalkyl oder das Phenylalkyl, das durch eine bis drei Halogen-, -R₄-, -OR₅-, -N(R₅)₂-, -COR₅-, -COOR₅-, -OCOR₅-, -CN-, -NO₂-, -SR₅-, -SOR₅-, -SO₂R₅- oder -P(O)(OR₅)₂-, Morpholinyl-, Piperidinyl-, 2,2,6,6-Tetramethylpiperidinyl, Piperazinyl oder N-Methylpiperidinylgruppen oder Kombinationen davon substituiert ist; oder das Alkyl oder das Cycloalkyl, das durch eine bis vier Phenylen-, -O-, -NR₅-, -CONR₅-, -COO-, -OCO- oder -CO-Gruppen oder Kombinationen davon unterbrochen ist; oder das Alkyl oder das Cycloalkyl, das durch Kombinationen der oben aufgeführten Gruppen sowohl substituiert als auch unterbrochen ist, steht;
R₁' R₁' und R₁" gleich oder verschieden sind und wie für R₁ definiert sind;
R₂ für Wasserstoff, gerad- oder verzweigtkettiges Alkyl mit 1 bis 24 Kohlenstoffatomen oder Cycloalkyl mit 5 bis 12 Kohlenstoffatomen oder das Alkyl oder das Cycloalkyl, das durch ein bis vier Halogen, Epoxy, Glycidyloxy, Furyloxy, -R₄, -OR₅, -N(R₅)₂, -CON(R₅)₂, -COR₅, -COOR₅, -OCOR₅, -OCOC(R₅)=C(R₅)₂, -C(R₅)=CCOOR₅, -CN, -NCO oder 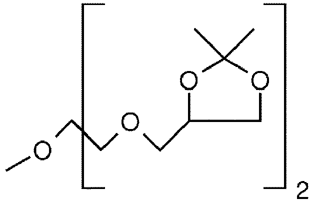 oder Kombinationen davon substituiert ist; oder das Alkyl oder das Cycloalkyl, das durch ein bis vier Epoxy, -O-, -NR₅-, -CONR₅-, -COO-, -OCO-, -CO-, -C(R₅)=C(R₅)COO-, -OCOC(R₅)=C(R₅)-, -C(R₅)=C(R₅)-, Phenylen oder Phenylen-G-phenylen unterbrochen ist, wobei G für -O-, -S-, -SO₂-, -CH₂- oder -C(CH₃)₂- oder Kombinationen davon steht, oder das Alkyl oder das Cycloalkyl, das durch Kombinationen der oben aufgeführten Gruppen sowohl substituiert als auch unterbrochen ist, steht oder R₂ für -SO₂R₃ oder -COR₆ steht;
R₂, R₂' und R₂" gleich oder verschieden sind und wie für R₂ definiert sind;
R₃ für Alkyl mit 1 bis 20 Kohlenstoffatomen, Alkenyl mit 3 bis 18 Kohlenstoffatomen, Cycloalkyl mit 5 bis 12 Kohlenstoffatomen, Phenylalkyl mit 7 bis 15 Kohlenstoffatomen, Aryl mit 6 bis 10 Kohlenstoffatomen oder das Aryl, das durch ein oder zwei Alkyl mit 1 bis 4 Kohlenstoffatomen substituiert ist, steht;
R₄ für Aryl mit 6 bis 10 Kohlenstoffatomen oder das Aryl, das durch ein bis drei Halogen, Alkyl mit 1 bis 8 Kohlenstoffatomen, Alkoxy mit 1 bis 8 Kohlenstoffatomen oder Kombinationen davon substituiert ist; Cycloalkyl mit 5 bis 12 Kohlenstoffatomen; Phenylalkyl mit 7 bis 15 Kohlenstoffatomen oder das Phenylalkyl, das am Phenylring durch ein bis drei Halogen, Alkyl mit 1 bis 8 Kohlenstoffatomen, Alkoxy mit 1 bis 8 Kohlenstoffatomen oder Kombinationen davon substituiert ist; oder gerad- oder verzweigtkettiges Alkenyl mit 2 bis 18 Kohlenstoffatomen steht;
R₅ wie für R₄ definiert ist oder R₅ auch für Wasserstoff oder gerad- oder verzweigtkettiges Alkyl mit 1 bis 24 Kohlenstoffatomen oder Alkenyl mit 2 bis 24 Kohlenstoffatomen steht oder R₅ für eine Gruppe der Formel 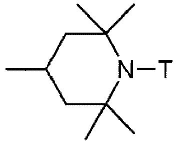 steht;
T für Wasserstoff, Oxyl, Hydroxyl, -OT₁, Alkyl mit 1 bis 24 Kohlenstoffatomen, das Alkyl, das durch ein bis drei Hydroxy substituiert ist; Benzyl oder Alkanoyl mit 2 bis 18 Kohlenstoffatomen steht;
T₁ für Alkyl mit 1 bis 24 Kohlenstoffatomen, Cycloalkyl mit 5 bis 12 Kohlenstoffatomen, Alkenyl mit 2 bis 24 Kohlenstoffatomen, Cycloalkenyl mit 5 bis 12 Kohlenstoffatomen, Phenylalkyl mit 7 bis 15 Kohlenstoffatomen, einen Rest eines gesättigten oder ungesättigten bicyclischen oder tricyclischen Kohlenwasserstoffs mit 7 bis 12 Kohlenstoffatomen oder Aryl mit 6 bis 10 Kohlenstoffatomen oder das Aryl, das durch ein bis drei Alkyl mit 1 bis 4 Kohlenstoffatomen substituiert ist, steht;
R₆ für gerad- oder verzweigtkettiges Alkyl mit 1 bis 18 Kohlenstoffatomen, gerad- oder verzweigtkettiges Alkenyl mit 2 bis 12 Kohlenstoffatomen, Phenoxy, Alkylamino mit 1 bis 12 Kohlenstoffatomen, Arylamino mit 6 bis 12 Kohlenstoffatomen, -R₇COOH oder -NH-R₈-NCO steht;
R₇ für Alkylen mit 2 bis 14 Kohlenstoffatomen oder Phenylen steht;
R₈ für Alkylen mit 2 bis 24 Kohlenstoffatomen, Phenylen, Tolylen, Diphenylmethan oder eine Gruppe 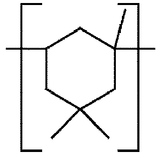 steht;
t für 0 bis 9 steht;
L für gerades oder verzweigtes Alkylen mit 1 bis 12 Kohlenstoffatomen, Cycloalkylen mit 5 bis 12 Kohlenstoffatomen oder Alkylen, das substituiert oder durch Cyclohexylen oder Phenylen unterbrochen ist, steht oder L für Benzyliden steht oder L für -S-, -S-S-, -S-E-S-, -SO-, -SO₂-, -SO-E-SO-, -SO₂-E-SO₂-, -CH₂-NH-E-NH-CH₂- oder 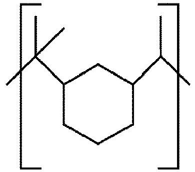 steht;
E für Alkylen mit 2 bis 12 Kohlenstoffatomen, Cycloalkylen mit 5 bis 12 Kohlenstoffatomen oder Alkylen, das durch Cycloalkylen mit 5 bis 12 Kohlenstoffatomen unterbrochen oder terminiert ist, steht;
n für 2, 3 oder 4 steht;
dann, wenn n für 2 steht, Q für gerades oder verzweigtes Alkylen mit 2 bis 16 Kohlenstoffatomen oder das Alkylen, das durch eine bis drei Hydroxygruppen substituiert ist; oder das Alkylen, das durch ein bis drei -CH=CH- oder -O-unterbrochen ist; oder das Alkylen, das durch Kombinationen der oben aufgeführten Gruppen sowohl substituiert als auch unterbrochen ist; steht oder Q für Xylylen oder eine Gruppe -CONH-R₈-NHCO-, -CH₂CH(OH)CH₂O-R₉-OCH₂CH(OH)CH₂-, -CO-R₁₀-CO- oder -(CH₂)ₘ-COO-R₁₁-OOC-(CH₂)ₘ- steht, wobei m für 1 bis 3 steht; oder Q für 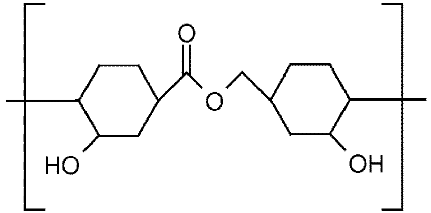 steht;
R₉ für Alkylen mit 2 bis 50 Kohlenstoffatomen oder das Alkylen, das durch ein bis zehn -O-, Phenylen oder eine Gruppe-Phenylen-G-phenylen, in der G für -O-, -S-, -SO₂-, -CH₂- oder -C(CH₃)₂- steht, unterbrochen ist, steht;
R₁₀ für Alkylen mit 2 bis 10 Kohlenstoffatomen oder das Alkylen, das durch ein bis vier -O-, -S- oder -CH=CH- unterbrochen ist, steht oder R₁₀ für Arylen mit 6 bis 12 Kohlenstoffatomen steht;
R₁₁ für Alkylen mit 2 bis 20 Kohlenstoffatomen oder das Alkylen, das durch ein bis acht -O-unterbrochen ist, steht;
dann, wenn n für 3 steht, Q für eine Gruppe -[(CH₂)ₘCOO]₃-R₁₂ steht, wobei m für 1 bis 3 steht und R₁₂ für ein Alkantriyl mit 3 bis 12 Kohlenstoffatomen steht;
dann, wenn n für 4 steht, Q für eine Gruppe -[(CH₂)ₘCOO]₄-R₁₃ steht, wobei m für 1 bis 3 steht und R₁₃ für ein Alkantetrayl mit 4 bis 12 Kohlenstoffatomen steht;
Z₁ für eine Gruppe der Formel 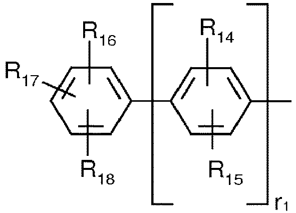 steht;
Z₂ für eine Gruppe der Formel 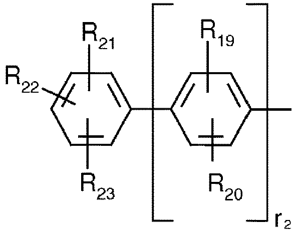 steht;
wobei
r₁ und r₂ unabhängig voneinander für 0 oder 1 stehen;
R₁₄, R₁₅, R₁₆, R₁₇, R₁₈, R₁₉, R₂₀, R₂₁, R₂₂ und R₂₃ unabhängig voneinander für Wasserstoff, Hydroxy, Cyano, Alkyl mit 1 bis 20 Kohlenstoffatomen, Alkoxy mit 1 bis 20 Kohlenstoffatomen, Phenylalkyl mit 7 bis 15 Kohlenstoffatomen, Cycloalkyl mit 5 bis 12 Kohlenstoffatomen, Cycloalkoxy mit 5 bis 12 Kohlenstoffatomen, Halogen, Halogenalkyl mit 1 bis 5 Kohlenstoffatomen, Sulfo, Carboxy, Acylamino mit 2 bis 12 Kohlenstoffatomen, Acyloxy mit 2 bis 12 Kohlenstoffatomen, Alkoxycarbonyl mit 2 bis 12 Kohlenstoffatomen oder Aminocarbonyl stehen oder R₁₇ und R₁₈ oder R₂₂ und R₂₃ zusammen mit dem Phenylrest, an den sie gebunden sind, einen cyclischen Rest, der durch ein bis drei -O- oder -NR₅- unterbrochen ist, bilden.

3. Getönte Klarlackzusammensetzung nach Anspruch 2, wobei es sich bei Komponente (a) um einen 2-Hydroxyphenyltriazin-UV-Absorber der Formel (I), (II) oder (III) handelt, wobei
X und Y unabhängig für Phenyl oder das Phenyl, das durch ein bis drei Alkyl mit 1 bis 6 Kohlenstoffatomen, durch Hydroxy oder durch Alkoxy mit 1 bis 6 Kohlenstoffatomen oder durch Mischungen davon substituiert ist, oder unabhängig für Z₁ oder Z₂ stehen;
R₁ für Wasserstoff steht;
R₁' und R₁" wie für R₁ definiert sind;
R₂ für Wasserstoff, gerad- oder verzweigtkettiges Alkyl mit 1 bis 24 Kohlenstoffatomen oder das Alkyl, das durch ein bis vier -R₄, -OR₅, -N(R₅)₂, -CON(R₅)₂, -COR₅, -COOR₅, -OCOR₅, -OCOC(R₅)=C(R₅)₂, -C(R₅)=CCOOR₅ oder Kombinationen davon substituiert ist, oder das Alkyl, das durch ein bis vier -O-, - NR₅-, -CONR₅-, -COO-, -OCO-, -CO-, -(C(R₅)=C(R₅)COO-, -OCOC(R₅)=C(R₅)-, -C(R₅)=C(R₅)- oder Kombinationen davon unterbrochen ist, oder das Alkyl, das durch Kombinationen der oben aufgeführten Gruppen sowohl substituiert als auch unterbrochen ist, steht oder R₂ für -COR₆ steht;
R₂, R₂' und R₂" gleich oder verschieden sind und wie für R₂ definiert sind;
R₄ für gerad- oder verzweigtkettiges Alkenyl mit 2 bis 18 Kohlenstoffatomen steht;
R₅ wie für R₄ definiert ist oder R₅ auch für Wasserstoff oder gerad- oder verzweigtkettiges Alkyl mit 1 bis 24 Kohlenstoffatomen oder Alkenyl mit 2 bis 24 Kohlenstoffatomen steht;
R₆ für gerad- oder verzweigtkettiges Alkyl mit 1 bis 18 Kohlenstoffatomen, gerad- oder verzweigtkettiges Alkenyl mit 2 bis 12 Kohlenstoffatomen oder Alkylamino mit 1 bis 12 Kohlenstoffatomen steht;
Z₁ für eine Gruppe der Formel 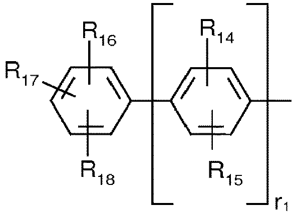 steht;
Z₂ für eine Gruppe der Formel 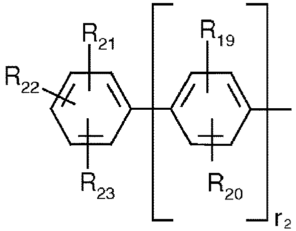 steht;
wobei
r₁ und r₂ unabhängig voneinander für 0 oder 1 stehen;
R₁₄, R₁₅, R₁₆, R₁₇, P₁₈, R₁₉, R₂₀, R₂₁, R₂₂ und R₂₃ unabhängig voneinander für Wasserstoff, Hydroxy, Alkyl mit 1 bis 20 Kohlenstoffatomen, Alkoxy mit 1 bis 20 Kohlenstoffatomen, Carboxy, Acylamino mit 2 bis 12 Kohlenstoffatomen, Acyloxy mit 2 bis 12 Kohlenstoffatomen, Alkoxycarbonyl mit 2 bis 12 Kohlenstoffatomen oder Aminocarbonyl stehen.

4. Getönte Klarlackzusammensetzung nach einem der Ansprüche 1-3, wobei es sich bei Komponente (a) um eine Mischung von zwei oder mehr verschiedenen 2-Hydroxyphenyltriazin-UV-Absorbern handelt.

5. Getönte Klarlackzusammensetzung nach einem der Ansprüche 1-4, wobei es sich bei Komponente (b) um ein C.I. Pigment Red 170, 177, 179, 202, 254, 264, ein C.I. Pigment Violet 19, 23, ein C.I. Pigment Blue 15, 15:1, 15:2, 15:3, 15:4, 15:6, 16, 60, ein C.I. Pigment Yellow 109, 110, 129, 138, 139, 150, 184, ein C.I. Pigment Green 7, 36, ein C.I. Pigment Orange 48, 73, ein Diketopyrrolopyrrol-Pigment, ein Chinacridon-Pigment, ein Chinacridon/Diketopyrrolopyrrol-Pigment sowie Mischungen oder Mischkristalle davon handelt.

6. Getönte Klarlackzusammensetzung nach einem der Ansprüche 1-5, wobei es sich bei Komponente (c) um ein Alkydharz, ein Polyesterharz, ein Acrylharz, ein Epoxidharz, ein Polyurethanharz, ein Melamin/Formaldehyd-Harz, ein Harnstoff/Formaldehyd-Harz, ein blockiertes Isocyanatharz oder Kombinationen davon handelt.

7. Getönte Klarlackzusammensetzung nach einem der Ansprüche 1-6, wobei es sich bei der Lackzusammensetzung um eine Autolackzusammensetzung handelt.

8. Getönte Klarlackzusammensetzung nach einem der Ansprüche 1-7, wobei Komponente (a) in einer Menge von 0,2 bis 20 Gew.-% des festen Bindemittels (c) vorliegt.

9. Getönte Klarlackzusammensetzung nach einem der Ansprüche 1-8, wobei Komponente (b) in einer Menge von 0,2 bis 10 Gew.-% des festen Bindemittels (c) vorliegt.

10. Getönte Klarlackzusammensetzung nach einem der Ansprüche 1-9, die weitere Additive umfasst.

11. Getönte Klarlackzusammensetzung nach Anspruch 10, die als weitere Additive phenolische und/oder aminische Antioxidantien, sterisch gehinderte Aminstabilisatoren, UV-Absorber, die von denjenigen gemäß Anspruch 1 verschieden sind, Phosphite, Phosphonite, Benzofuranone, Metallstearate, Metalloxide, Organophosphorverbindungen, Hydroxylamine und/oder Flammschutzmittel umfasst.

12. Getönte Klarlackzusammensetzung nach Anspruch 11, die als weitere Additive sterisch gehinderte Aminstabilisatoren und/oder UV-Absorber aus der Gruppe bestehend aus den Oxamiden, den 2-Hydroxybenzophenonen, den Benzoaten, den Acrylaten und den 2-(2'-Hydroxyphenyl)benzotriazolen umfasst.

13. Getönter Klarlack, erhalten durch Aufbringen einer getönten Klarlackzusammensetzung nach einem der Ansprüche 1-12 auf ein Substrat.

14. Getönter Klarlack nach Anspruch 13, bei dem es sich um einen Autolack handelt.

15. Getönter Klarlack nach Anspruch 14, wobei der Autolack die folgenden Schichten umfasst:
(d) einen kathodisch abgeschiedenen Lack, der auf einem Metallsubstrat haftet;
(e) mindestens eine darauffolgende Lackschicht, die auf dem kathodisch abgeschiedenen Lack haftet;
(f) mindestens eine Basislackschicht, die ein Pigment oder Pigmentmischungen enthält;
(g) einen getönten Klarlack gemäß Anspruch 13.

16. Verfahren zur Herstellung eines getönten Klarlacks, bei dem man eine getönte Klarlackzusammensetzung gemäß einem der Ansprüche 1-12 auf ein Substrat aufbringt.

17. Verfahren nach Anspruch 16, bei dem das Substrat die folgenden Schichten umfasst:
(d) einen kathodisch abgeschiedenen Lack, der auf einem Metallsubstrat haftet;
(e) mindestens eine darauffolgende Lackschicht, die auf dem kathodisch abgeschiedenen Lack haftet; und
(f) mindestens eine Basislackschicht, die ein Pigment oder Pigmentmischungen enthält.

## Revendications

1. Composition de revêtement transparent teinté comprenant
(a) au moins un absorbeur d'UV 2-hydroxyphényltriazine,
(b) au moins un pigment choisi dans le groupe constitué par les pigments quinacridone, dicétopyrrolopyrrole, quinacridone/dicétopyrrolopyrrole, isoindolinone, phtalocyanine, pérylène, rouge d'anthraquinone, bleu d'indanthrone, complexe de Cu et d'azométhine, complexe de Ni monoazoïque, quinophtalone, isoindoline et naphtol AS et les mélanges et solutions solides de ceux-ci, le rapport du composant (a) au composant (b) étant de 10:1 à 1:1 en poids, et
(c) au moins un liant.

2. Compositions de revêtement transparent teinté selon la revendication 1, dans lesquelles l'absorbeur d'UV 2-hydroxyphényltriazine répond à la formule (I), (II), (III), (IV), (V) ou (VI) 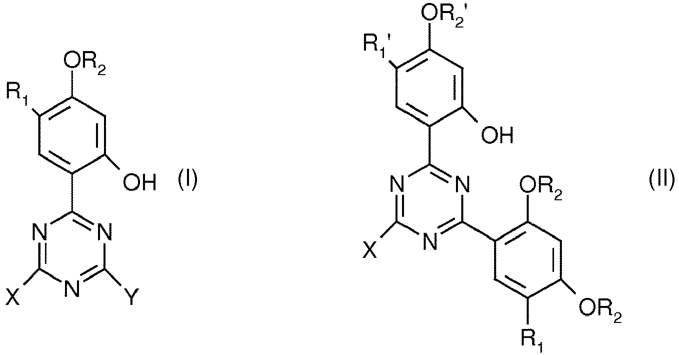 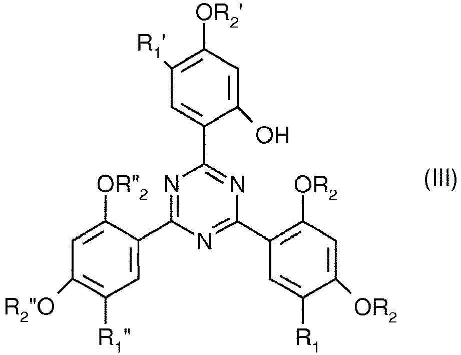 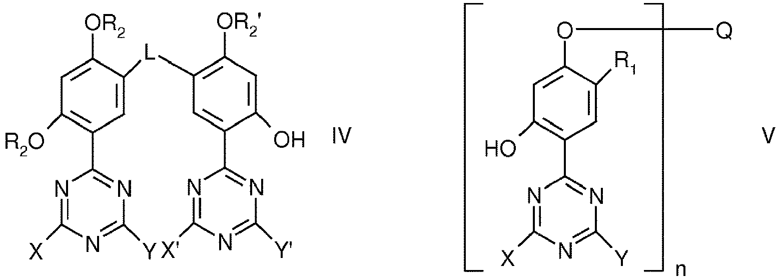 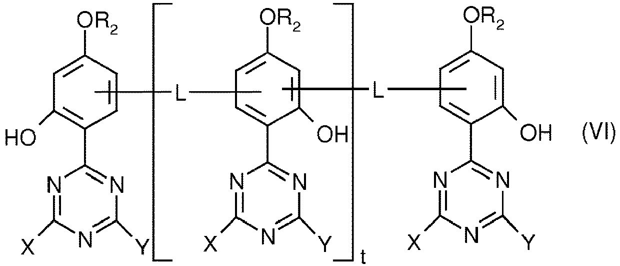 dans lesquelles
X et Y sont chacun indépendamment un groupe phényle, un groupe naphtyle, un groupe pyrényle, un groupe phénanthrényle ou un groupe fluoranthényle ou ledit groupe phényle, ledit groupe naphtyle, ledit groupe pyrényle, ledit groupe phénanthrényle ou ledit groupe fluoranthényle substitué par un à trois groupes alkyle de 1 à 6 atomes de carbone, par un atome d'halogène, par un groupe hydroxy ou par un groupe alcoxy de 1 à 6 atomes de carbone ou par des mélanges de ceux-ci ; ou sont indépendamment Z₁ ou Z₂ ;
X, X', Y et Y' sont identiques ou différents et sont tels que définis pour X et Y ;
R₁ est l'atome d'hydrogène, un groupe alkyle de 1 à 24 atomes de carbone à chaîne droite ou ramifiée, un groupe cycloalkyle de 5 à 12 atomes de carbone, un groupe phénylalkyle de 7 à 15 atomes de carbone, un atome d'halogène, -SR₃, -SOR₃ ou -SO₂R₃ ; ou ledit groupe alkyle, ledit groupe cycloalkyle ou ledit groupe phénylalkyle substitué par un à trois groupes halogéno, -R₄, -OR₅, -N(R₅)₂, -COR₅, -COOR₅, -OCOR₅, -CN, -NO₂, -SR₅, -SOR₅, -SO₂R₅ ou -P(O)(OR₅)₂, morpholinyle, pipéridinyle, 2,2,6,6-tétraméthylpipéridinyle, pipérazinyle ou N-méthylpipéridinyle ou des combinaisons de ceux-ci ; ou ledit groupe alkyle ou ledit groupe cycloalkyle interrompu par un à quatre groupes phénylène, -O-, -NR₅-, -CONR₅-, -COO-, -OCO- ou -CO- ou des combinaisons de ceux-ci ; ou ledit groupe alkyle ou ledit groupe cycloalkyle à la fois substitué et interrompu par des combinaisons des groupes mentionnés ci-dessus ;
R₁, R₁' et R₁" sont identiques ou différents et sont tels que définis pour R₁ ;
R₂ est l'atome d'hydrogène, un groupe alkyle de 1 à 24 atomes de carbone à chaîne droite ou ramifiée ou un groupe cycloalkyle de 5 à 12 atomes de carbone ; ou ledit groupe alkyle ou ledit groupe cycloalkyle substitué par un à quatre groupes halogéno, époxy, glycidyloxy, furyloxy, -R₄, -OR₅, -N(R₅)₂, -CON(R₅)₂, -COR₅, -COOR₅, -OCOR₅, -OCOC(R₅)=C(R₅)₂, -C(R₅)=CCOOR₅, -CN, -NCO ou 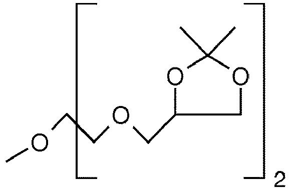 ou des combinaisons de ceux-ci ; ou ledit groupe alkyle ou ledit groupe cycloalkyle interrompu par un à quatre groupes époxy, -O-, -NR₅-, -CONR₅-, -COO-, -OCO-, -CO-, -C(R₅)=C(R₅)COO-, -OCOC(R₅)=C(R₅)-, -C(R₅)=C(R₅)-, phénylène ou phénylène-G-phénylène, G étant -O-, -S-, -SO₂-, -CH₂- ou -C(CH₃)₂- ou des combinaisons de ceux-ci, ou ledit groupe alkyle ou ledit groupe cycloalkyle à la fois substitué et interrompu par des combinaisons des groupes mentionnés ci-dessus ; ou R₂ est -SO₂R₃ ou -COR₆ ;
R₂, R₂' et R₂" sont identiques ou différents et sont tels que définis pour R₂ ;
R₃ est un groupe alkyle de 1 à 20 atomes de carbone, un groupe alcényle de 3 à 18 atomes de carbone, un groupe cycloalkyle de 5 à 12 atomes de carbone, un groupe phénylalkyle de 7 à 15 atomes de carbone, un groupe aryle de 6 à 10 atomes de carbone ou ledit groupe aryle substitué par un ou deux groupes alkyle de 1 à 4 atomes de carbone ;
R₄ est un groupe aryle de 6 à 10 atomes de carbone ou ledit groupe aryle substitué par un à trois groupes halogéno, alkyle de 1 à 8 atomes de carbone, alcoxy de 1 à 8 atomes de carbone ou des combinaisons de ceux-ci ; un groupe cycloalkyle de 5 à 12 atomes de carbone ; un groupe phénylalkyle de 7 à 15 atomes de carbone ou ledit groupe phénylalkyle substitué sur le noyau phényle par un à trois groupes halogéno, alkyle de 1 à 8 atomes de carbone, alcoxy de 1 à 8 atomes de carbone ou des combinaisons de ceux-ci ; ou un groupe alcényle de 2 à 18 atomes de carbone à chaîne droite ou ramifiée ;
R₅ est défini comme l'est R₄ ; ou R₅ est également l'atome d'hydrogène ou un groupe alkyle de 1 à 24 atomes de carbone à chaîne droite ou ramifiée, un groupe alcényle de 2 à 24 atomes de carbone ; ou R₅ est un groupe de formule 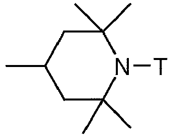
T est l'atome d'hydrogène, un groupe oxyle, un groupe hydroxyle, -OT₁, un groupe alkyle de 1 à 24 atomes de carbone, ledit groupe alkyle substitué par un à trois groupes hydroxy ; un groupe benzyle ou un groupe alcanoyle de 2 à 18 atomes de carbone ;
T₁ est un groupe alkyle de 1 à 24 atomes de carbone, un groupe cycloalkyle de 5 à 12 atomes de carbone, un groupe alcényle de 2 à 24 atomes de carbone, un groupe cycloalcényle de 5 à 12 atomes de carbone, un groupe phénylalkyle de 7 à 15 atomes de carbone, un radical d'un hydrocarbure bicyclique ou tricyclique saturé ou insaturé de 7 à 12 atomes de carbone ou un groupe aryle de 6 à 10 atomes de carbone ou ledit groupe aryle substitué par un à trois groupes alkyle de 1 à 4 atomes de carbone ;
R₆ est un groupe alkyle de 1 à 18 atomes de carbone à chaîne droite ou ramifiée, un groupe alcényle de 2 à 12 atomes de carbone à chaîne droite ou ramifiée, un groupe phénoxy, un groupe alkylamino de 1 à 12 atomes de carbone, un groupe arylamino de 6 à 12 atomes de carbone, -R₇COOH ou -NH-R₈-NCO ;
R₇ est un groupe alkylène de 2 à 14 atomes de carbone ou un groupe phénylène ;
R₈ est un groupe alkylène de 2 à 24 atomes de carbone, un groupe phénylène, un groupe tolylène, un groupe diphénylméthane ou un groupe 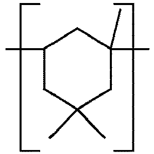
t vaut 0 à 9 ;
L est un groupe alkylène de 1 à 12 atomes de carbone droit ou ramifié, un groupe cycloalkylène de 5 à 12 atomes de carbone ou un groupe alkylène substitué ou interrompu par un groupe cyclohexylène ou phénylène ; ou L est un groupe benzylidène ; ou L est -S-, -S-S-, -S-E-S-, -SO-, -SO₂-, -SO-E-SO-, -SO₂-E-SO₂-, -CH₂-NH-E-NH-CH₂- ou 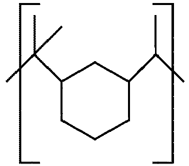
E est un groupe alkylène de 2 à 12 atomes de carbone, un groupe cycloalkylène de 5 à 12 atomes de carbone ou un groupe alkylène interrompu ou terminé par un groupe cycloalkylène de 5 à 12 atomes de carbone ;
n vaut 2, 3 ou 4 ;
lorsque n vaut 2 ; Q est un groupe alkylène de 2 à 16 atomes de carbone droit ou ramifié ; ou ledit groupe alkylène substitué par un à trois groupes hydroxy ; ou ledit groupe alkylène interrompu par un à trois groupes -CH=CH- ou -O- ; ou ledit groupe alkylène à la fois substitué et interrompu par des combinaisons des groupes mentionnés ci-dessus ; ou Q est un groupe xylylène ou un groupe -CONH-R₈-NHCO-, -CH₂CH(OH)CH₂O-R₉-OCH₂CH(OH)CH₂-, -CO-R₁₀-CO- ou -(CH₂)ₘ-COO-R₁₁-OOC-(CH₂)ₘ-, m valant 1 à 3 ; ou Q est 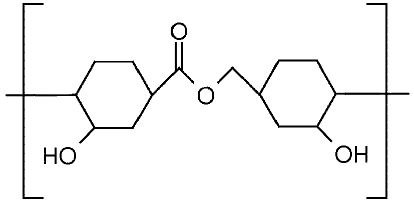
R₉ est un groupe alkylène de 2 à 50 atomes de carbone ; ou ledit groupe alkylène interrompu par un à dix groupes -O-, un groupe phénylène ou un groupe -phénylène-G-phénylène dans lequel G est -O-, -S-, -SO₂-, -CH₂- ou -C(CH₃)₂- ;
R₁₀ est un groupe alkylène de 2 à 10 atomes de carbone ou ledit groupe alkylène interrompu par un à quatre groupes -O-, -S- ou -CH=CH- ; ou R₁₀ est un groupe arylène de 6 à 12 atomes de carbone ;
R₁₁ est un groupe alkylène de 2 à 20 atomes de carbone ou ledit groupe alkylène interrompu par un à huit groupes -O- ;
lorsque n vaut 3, Q est un groupe -[(CH₂)ₘCOO]₃-R₁₂, m valant 1 à 3 et R₁₂ étant un groupe alcanetriyle de 3 à 12 atomes de carbone ; lorsque n vaut 4, Q est un groupe -[(CH₂)ₘCOO]₄-R₁₃, m valant 1 à 3 et R₁₃ étant un groupe alcanetétrayle de 4 à 12 atomes de carbone ;
Z₁ est un groupe de formule 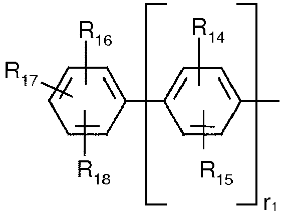
Z₂ est un groupe de formule 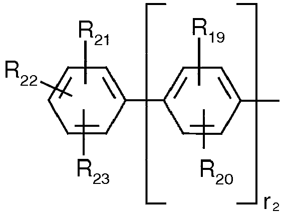
où
r₁ et r₂ valent chacun indépendamment de l'autre 0 ou 1 ;
R₁₄, R₁₅, R₁₆, R₁₇, R₁₈, R₁₉, R₂₀, R₂₁, R₂₂, et R₂₃ sont chacun indépendamment des autres l'atome d'hydrogène, un groupe hydroxy, un groupe cyano, un groupe alkyle de 1 à 20 atomes de carbone, un groupe alcoxy de 1 à 20 atomes de carbone, un groupe phénylalkyle de 7 à 15 atomes de carbone, un groupe cycloalkyle de 5 à 12 atomes de carbone, un groupe cycloalcoxy de 5 à 12 atomes de carbone, un atome d'halogène, un groupe halogénoalkyle de 1 à 5 atomes de carbone, un groupe sulfo, un groupe carboxy, un groupe acylamino de 2 à 12 atomes de carbone, un groupe acyloxy de 2 à 12 atomes de carbone, un groupe alcoxycarbonyle de 2 à 12 atomes de carbone ou un groupe aminocarbonyle ; ou R₁₇ et R₁₈ ou R₂₂ et R₂₃ conjointement avec le radical phényle auquel ils sont attachés forment un radical cyclique interrompu par un à trois groupes -O- ou -NR₅-.

3. Composition de revêtement transparent teinté selon la revendication 2, dans laquelle le composant (a) est un absorbeur d'UV 2-hydroxyphényltriazine de formule (I), (II) ou (III), dans lesquelles
X et Y sont indépendamment un groupe phényle ou ledit groupe phényle substitué par un à trois groupes alkyle de 1 à 6 atomes de carbone, par un groupe hydroxy ou par un groupe alcoxy de 1 à 6 atomes de carbone ou par des mélanges de ceux-ci ; ou sont indépendamment Z₁ ou Z₂ ;
R₁ est l'atome d'hydrogène ;
R₁' et R₁" sont tels que définis pour R₁ ;
R₂ est l'atome d'hydrogène, un groupe alkyle de 1 à 24 atomes de carbone à chaîne droite ou ramifiée ; ou ledit groupe alkyle substitué par un à quatre groupes -R₄, -OR₅, -N(R₅)₂, -CON(R₅)₂, -COR₅, -COOR₅, -OCOR₅, -OCOC(R₅)=C(R₅)₂, -C(R₅)=CCOOR₅ ou des combinaisons de ceux-ci ; ou ledit groupe alkyle interrompu par un à quatre groupes -O-, -NR₅-, -CONR₅-, -COO-, -OCO-, -CO-, -C(R₅)=C(R₅)COO-, -OCOC(R₅)=C(R₅)-, -C(R₅)=C(R₅)- ou des combinaisons de ceux-ci ou ledit groupe alkyle à la fois substitué et interrompu par des combinaisons des groupes mentionnés ci-dessus ; ou R₂ est -COR₆ ;
R₂, R₂' et R₂" sont identiques ou différents et sont tels que définis pour R₂ ;
R₄ est un groupe alcényle de 2 à 18 atomes de carbone à chaîne droite ou ramifiée ;
R₅ est défini comme l'est R₄ ; ou R₅ est également l'atome d'hydrogène ou un groupe alkyle de 1 à 24 atomes de carbone à chaîne droite ou ramifiée, un groupe alcényle de 2 à 24 atomes de carbone ;
R₆ est un groupe alkyle de 1 à 18 atomes de carbone à chaîne droite ou ramifiée, un groupe alcényle de 2 à 12 atomes de carbone à chaîne droite ou ramifiée, un groupe alkylamino de 1 à 12 atomes de carbone ;
Z₁ est un groupe de formule 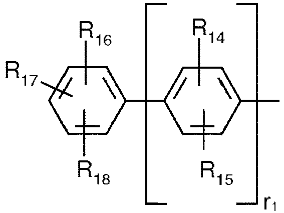
Z₂ est un groupe de formule 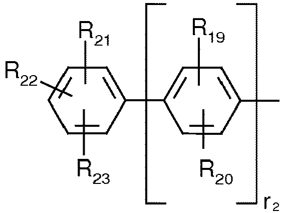
où
r₁ et r₂ valent chacun indépendamment de l'autre 0 ou 1 ;
R₁₄, R₁₅, R₁₆, R₁₇, R₁₈, R₁₉, R₂₀, R₂₁, R₂₂ et R₂₃ sont chacun indépendamment des autres l'atome d'hydrogène, un groupe hydroxy, un groupe alkyle de 1 à 20 atomes de carbone, un groupe alcoxy de 1 à 20 atomes de carbone, un groupe carboxy, un groupe acylamino de 2 à 12 atomes de carbone, un groupe acyloxy de 2 à 12 atomes de carbone, un groupe alcoxycarbonyle de 2 à 12 atomes de carbone ou un groupe aminocarbonyle.

4. Composition de revêtement transparent teinté selon l'une quelconque des revendications 1-3, dans laquelle le composant (a) est un mélange de deux ou plus de deux absorbeurs d'UV 2-hydroxyphényltriazines différents.

5. Composition de revêtement transparent teinté selon la revendication 1-4, dans laquelle le composant (b) est un C.I. pigment rouge 170, 177, 179, 202, 254, 264, un C.I. pigment violet 19, 23, un C.I. pigment bleu 15, 15:1, 15:2, 15:3, 15:4, 15:6, 16, 60, un C.I. pigment jaune 109, 110, 129, 138, 139, 150, 184, un C.I. pigment vert 7, 36, un C.I. pigment orange 48, 73, un pigment dicétopyrrolopyrrole, un pigment quinacridone, un pigment quinacridone/dicétopyrrolopyrrole ; ainsi que des mélanges ou solutions solides de ceux-ci.

6. Composition de revêtement transparent teinté selon l'une quelconque des revendications 1-5, dans laquelle le composant (c) est une résine alkyde, une résine de polyester, une résine acrylique, une résine époxyde, une résine de polyuréthane, une résine mélamine/formaldéhyde, une résine urée/formaldéhyde, une résine d'isocyanate bloqué ou des combinaisons de celles-ci.

7. Composition de revêtement transparent teinté selon l'une quelconque des revendications 1-6, la composition de revêtement étant une composition de revêtement pour automobile.

8. Composition de revêtement transparent teinté selon l'une quelconque des revendications 1-7, dans laquelle le composant (a) est présent en une quantité de 0,2 à 20 % en poids du liant solide (c).

9. Composition de revêtement transparent teinté selon l'une quelconque des revendications 1-8, dans laquelle le composant (b) est présent en une quantité de 0,2 à 10 % en poids du liant solide (c).

10. Composition de revêtement transparent teinté selon l'une quelconque des revendications 1-9, qui comprend d'autres additifs.

11. Composition de revêtement transparent teinté selon la revendication 10, qui comprend en tant qu'autres additifs des antioxydants phénoliques et/ou aminofonctionnels, des stabilisants amines stériquement encombrées, des absorbeurs d'UV différents de ceux tels que définis dans la revendication 1, des phosphites, des phosphonites, des benzofuranones, des stéarates métalliques, des oxydes métalliques, des composés organophosphorés, des hydroxylamines et/ou des agents ignifugeants.

12. Composition de revêtement transparent teinté selon la revendication 11, qui comprend en tant qu'autres additifs des stabilisants amines stériquement encombrées et/ou des absorbeurs d'UV choisis dans le groupe constitué par les oxamides, les 2-hydroxybenzophénones, les benzoates, les acrylates et les 2-(2'-hydroxyphényl)benzotriazoles.

13. Revêtement transparent teinté obtenu par application d'une composition de revêtement transparent teinté de l'une quelconque des revendications 1-12 sur un substrat.

14. Revêtement transparent teinté selon la revendication 13 qui est un revêtement pour automobile.

15. Revêtement transparent teinté selon la revendication 14, le revêtement pour automobile comprenant les couches suivantes
(d) un revêtement déposé par voie cathodique, adhérant à un substrat métallique ;
(e) au moins une couche de revêtement subséquente adhérant au revêtement déposé par voie cathodique ;
(f) au moins une couche de revêtement de base contenant un pigment ou des mélanges de pigments ;
(g) un revêtement transparent teinté tel que défini dans la revendication 13.

16. Procédé pour la préparation d'un revêtement transparent teinté qui comprend l'application d'une composition de revêtement transparent teinté selon l'une quelconque des revendications 1-12 sur un substrat.

17. Procédé selon la revendication 16, dans lequel le substrat comprend les couches suivantes
(d) un revêtement déposé par voie cathodique, adhérant à un substrat métallique ;
(e) au moins une couche de revêtement subséquente adhérant au revêtement déposé par voie cathodique ; et
(f) au moins une couche de revêtement de base contenant un pigment ou des mélanges de pigments.
